# EUROPÄISCHE PATENTSCHRIFT

(11) **EP 3 638 095 B1**
(45) Veröffentlichungstag und Bekanntmachung des Hinweises auf die Patenterteilung: **07.06.2023**
(21) Anmeldenummer: 18731402.6
(22) Anmeldetag: 12.06.2018
(51) Int. Cl.: A61B 3/11

(54) **VERFAHREN ZUM HOCHGENAUEN BESTIMMEN EINES PUPILLENDURCHMESSERS EINES AUGES UND EINE DIESBEZÜGLICHE VORRICHTUNG**
METHOD FOR DETERMINING THE PUPIL DIAMETER OF AN EYE WITH HIGH ACCURACY, AND CORRESPONDING APPARATUS
PROCÉDÉ DE DÉTERMINATION TRÈS PRÉCISE DU DIAMÈTRE DE LA PUPILLE D'UN OEIL ET DISPOSITIF CORRESPONDANT

(30) Priorität: 12.06.2017 LU 100287
(43) Veröffentlichungstag der Anmeldung: 22.04.2020
(73) Patentinhaber: Rodenstock GmbH, 80687 München (DE)
(72) Erfinder: SEITZ, Peter, 71272 Renningen (DE); TIEMANN, Markus, 81539 München (DE)
(74) Vertreter: RCD Patent Giesen, Schmelcher & Griebel Patentwanwälte PartG mbB
(86) Internationale Anmeldenummer: PCT/EP2018/065425
(87) Internationale Veröffentlichungsnummer: WO 2018/229021

(56) Entgegenhaltungen:
- DE-T2- 69 317 570
- US-A- 5 790 235
- NGUYEN A H ET AL: "Model control of image processing: Pupillometry", COMPUTERIZED MEDICAL IMAGING AND GRAPHICS, PERGAMON PRESS, NEW YORK, NY, US, Bd. 17, Nr. 1, 1. Januar 1993 (1993-01-01) , Seiten 21-33, XP022916353, ISSN: 0895-6111, DOI: 10.1016/0895-6111(93)90071-T [gefunden am 1993-01-01]
- Sebastiaan Mathôt ET AL: "The pupillary light response reveals the focus of covert visual attention", PLoS ONE, 1. Oktober 2013 (2013-10-01), XP055525058, DOI: 10.1371/journal.pone.0078168 Gefunden im Internet: URL:https://pdfs.semanticscholar.org/b761/ b39f5aa51e7691219aaa7de6b78b9c048c98.pdf

## Beschreibung

Die vorliegende Erfindung betrifft ein Verfahren zum hochgenauen Bestimmen eines Pupillendurchmessers eines Auges und eine diesbezügliche Vorrichtung.

### Hintergrund

Bei einer Ermittlung von Zentrierdaten für eine Brille ist es erforderlich die Blickrichtung des Probanden zu kennen. Dies kann durch Vorgabe eines Fixationsobjektes erfolgen über das vorab weitere Informationen bekannt sind oder bestimmt werden können, wie beispielsweise die Nasenwurzelmethode beim ImpressionIST, ein zusätzliches Referenzobjekt wie in US 7,909,460 B1 offenbart oder virtuell im unendlichen liegende Punkte.

Zudem können die individuellen Daten eine Position / einen Durchmesser einer Pupille umfassen der in der gleichen Situation gemessen wird und zur Optimierung der Gläser auf Basis von mittleren HOAs eingesetzt wird, wie in WO 2013 / 087 212 A1 offenbart.

Aus dem Artikel "Model control of image processing: Pupillometry" von NGUYEN A H ET AL. veröffentlicht in COMPUTERIZED MEDICAL IMAGING AND GRAPHICS, PERGAMON PRESS, NEWYORK, NY, US, vol. 17, no. 1, doi:10.1016/0895-6111(93)90071-T, ISSN 0895-6111, (19930101), pages 21 - 33, (19930101) ist ein Verfahren zur Bestimmung eines Pupillendurchmessers eines Auges bekannt.

Die gängigen Verfahren erfassen jedoch recht ungenau die Pupille beziehungsweise ermitteln den Pupillendurchmesser recht ungenau.

Ferner wird die Messung verschiedener einzelner Parameter durch unterschiedliche Geräteklassen ermöglicht. So gibt es Pupillometer zur Messung von Pupillendurchmessern, Eyetracker die eine Blickrichtungsmessung ermöglichen und Videozentriersysteme die Zentrierdaten ermitteln.

Die beiden erstgenannten Geräteklassen werden in der Regel unter Laborbedingungen eingesetzt, wobei Einschränkungen wie Kopffixierungen, Abwesenheit von Umgebungslicht, mangelnde Flexibilität durch Kabelverbindungen und am Probanden anzubringende Komponenten in Kauf genommen werden. Häufig ist zudem keine absolute Messung erforderlich sondern eine relative ausreichend, wie beispielsweise lediglich eine Pupillendurchmesser-Reaktion auf Lichtimpulse. Häufig kommen beim Eyetracking zudem Beleuchtungssysteme zum Einsatz, deren Reflex auf dem Auge analysiert wird.

Für die Videozentrierung wird bei der Bestimmung des Pupillendurchmessers üblicherweise der Blick nach unendlich zugrunde gelegt. Da es üblicherweise nicht möglich ist, ein Fixierobjekt im unendlichen anzubringen, werden Vorrichtungen benötigt, die entweder ein Objekt im unendlichen abbilden oder man nimmt eine Korrektur vor, die beim Blick auf ein bekanntes, in endlicher Entfernung positioniertes Objekt, die Differenz zum Pupillendurchmesser beim Blick nach unendlich bestimmen kann. Es kann jedoch dazu auch ein Augenmodell verwendet werden, insbesondere die Augenlänge ist dafür relevant. Dazu wird die Entfernung Auge Fixierobjekt benötigt. Beim ImpressionIST von Rodenstock wird als Fixierobjekt die Nasenwurzel des Spiegelbilds des Kunden verwendet.

Daher wäre es wünschenswert eine Möglichkeit bereitzustellen, mit dem der Pupillendurchmesser mit hoher Genauigkeit ermittelt werden kann, ohne eine Information über ein Fixationsobjekt bei einer Videozentrierung zu benötigen, somit einen Pupillendurchmesser präzise in einer gewöhnlichen Umgebung bestimmen zu können.

Es ist Ziel der Erfindung eine Möglichkeit vorzuschlagen, welche zumindest einen Teil der im Stand der Technik bekannten Nachteile vermeidet oder zumindest vermindert.

### Kurzdarstellung der Erfindung

Die Aufgabe wird erfindungsgemäß gelöst mittels eines Verfahrens gemäß dem Hauptanspruch, sowie mittels einer Vorrichtung gemäß einem nebengeordneten Anspruch.

Der Gegenstand des Hauptanspruches betrifft dabei ein Verfahren zum hochgenauen Bestimmen eines Pupillendurchmessers eines Auges, mittels eines Kamerasystems zum räumlichen Erfassen von Objekten, wobei das Verfahren aufweist: Bildgebendes stereoskopes Erfassen einer Augenregion eines Kopfes, mittels des Kamerasystems, wobei sich für ein Auge der Augenregion ein erstes und ein zweites stereoskopes Bild ergeben. Und für ein jedes der beiden stereoskopen Bilder weist das Verfahren ferner auf: Transferieren der erfassten Augenregion des Kopfes in einen Farbraum, und Weiterführen des Verfahrens in diesem Farbraum. Ermitteln eines Bereiches innerhalb der erfassten Augenregion, als einen Bereich innerhalb einer Pupille des Auges der erfassten Augenregion, wobei der ermittelte Bereich innerhalb der erfassten Augenregion ein Bildkriterium aufweist, wobei das Bildkriterium ein weitgehend homogenes Bildkriterium darstellt, wobei für ein weitgehend homogenes Bildkriterium eine Bildeigenschaft zumindest in einem signifikant großen Bereich des Bildes Homogenität aufweist. Ermitteln eines geschlossenen äußeren Randes des Bereiches innerhalb der Pupille. Iteratives Ermitteln eines geschlossenen äußeren Pupillenrandes, wobei das iterative Ermitteln des äußeren Pupillenrandes aufweist: Den äußeren Rand umlaufendes Ermitteln eines weiteren Bereiches außerhalb des Randes, entlang des Randes, wobei der weitere Bereich an den geschlossenen äußeren Rand des Bereiches innerhalb der Pupille angrenzt. Iteratives Untersuchen des weiteren Bereiches daraufhin, ob der weitere Bereich das Bildkriterium aufweist. Und falls zumindest ein Teil des weiteren Bereiches das Bildkriterium aufweist, erfolgt ein Integrieren desjenigen Teiles des weiteren Bereiches, welcher das Bildkriterium aufweist, in den Bereich innerhalb der Pupille des Auges und ein Ausweiten des geschlossenen äußeren Randes des Bereiches innerhalb der Pupille auf den integrierten Teil des weiteren Bereiches. Und falls der weitere Bereich das Bildkriterium vollständig nicht aufweist, erfolgt ein Festlegen des geschlossenen äußeren Randes des Bereiches innerhalb der Pupille als den äußeren Rand der Pupille des Auges. Das Verfahren weist für ein jedes der beiden stereoskopen Bilder ferner auf: Ermitteln eines dritten Bereiches als Pupille des Auges, wobei der dritte Bereich derjenige Bereich ist, der vollständig vom äußeren Rand der Pupille eingeschlossen ist.

In einer Ausführungsform weist das Verfahren weiterhin auf: Bestimmen eines Punktes in der Pupille. Bestimmen einer ersten Geraden durch den Punkt. Bestimmen einer zweiten Geraden durch den Punkt, wobei die zweite Gerade orthogonal zur ersten Geraden angeordnet ist. Und iteratives Ermitteln eines Pupillenmittelpunktes, wobei das iterative Ermitteln des Pupillenmittelpunktes aufweist: Ermitteln einer ersten Strecke und einer zweiten Strecke für jede Gerade, wobei das Ermitteln der jeweiligen Strecke aufweist: Ermitteln eines ersten Endpunktes der entsprechenden Strecke, wobei der erste Endpunkt dem Punkt in der Pupille entspricht. Ermitteln eines zweiten Endpunktes der entsprechenden Strecke, wobei der zweite Endpunkt einem Kreuzungspunkt der entsprechenden Geraden mit dem Rand der Pupille entspricht. Und ermitteln der entsprechenden Strecke, als eine Linie entlang der entsprechenden Geraden von dem entsprechenden ersten Endpunkt ausgehend zu dem entsprechenden zweiten Endpunkt hinführend. Ferner weist das iterative Ermitteln des Pupillenmittelpunktes auf: Iteratives längenmäßiges Vergleichen der beiden Strecken der ersten Geraden. Falls die beiden Strecken der ersten Geraden nicht gleich lang sind, erfolgt ein Inkrementieren einer ersten Koordinate des Punktes in der Pupille, entlang der längeren der beiden Strecken der ersten Geraden. Falls die beiden Strecken der zweiten Geraden nicht gleich lang sind, erfolgt ein Inkrementieren einer zweiten Koordinate des Punktes in der Pupille, entlang der längeren der beiden Strecken der zweiten Geraden. Und falls die beiden Strecken der ersten Geraden gleichlang sind und die beiden Strecken der zweiten Geraden gleichlang sind, erfolgt ein Festlegen des Punktes in der Pupille als den Pupillenmittelpunkt der Pupille des Auges.

In einer Ausführungsform weist das Verfahren weiterhin auf: Trianguliertes Überlagern der beiden Mittelpunkte der Pupillen des Auges der beiden stereoskopen Bilder. Trianguliertes Überlagern der beiden äußeren Ränder der Pupille des Auges der beiden stereoskopen Bilder. Und ermitteln des Pupillendurchmessers, basierend auf den beiden triangulierten Überlagerungen.

Die Verfahrensschritte können dabei jeweils automatisiert ausgeführt werden.

Eine Augenregion im Sinne der Erfindung meint dabei die Region des Gesichtes, in dem beide Augen liegen.

Ein Bildkriterium im Sinne der Erfindung kann dabei eine Eigenschaft eines Bildes sein, wie sie zur technischen Bildbeschreibung herangezogen wird. Solche Bildeigenschaften können beispielsweise ein Histogramm, Sättigung, Kontrast und dergleichen sein. Das Bildkriterium kann somit von dem genutzten Farbraum selbst abhängig sein.

Ein weitgehend homogenes Bildkriterium im Sinne der Erfindung meint dabei, dass die entsprechende Bildeigenschaft zumindest in einem signifikant großen Bereich des Bildes Homogenität aufweist. Auf eine Pupille bezogen kann solch ein weitgehend homogener Bereich innerhalb des ermittelten Bereiches innerhalb der erfassten Augenregion zumindest einen Teil der gesamten Pupillenfläche aufweisen beispielsweise mindestens 10% der Pupillenfläche, vorzugsweise mindestens 5% der Pupillenfläche.

Basierend auf einem Stereokamerasystem wird eine Aufnahme von einem Teilbereich eines Kopfes aufgenommen, so dass in beiden Aufnahmen die Augenregion sichtbar ist.

Davon abweichend kann anstatt der horizontalen Ausrichtung auch eine Ausrichtung der optischen Achse um einen bekannten Winkel erfolgen und dieser nachfolgend für die Videozentrierung berücksichtigt werden.

In beiden Aufnahmen wird die Pupille, vorzugsweise die Pupillenmitte selektiert.

Für die automatische Variante kommen Bildverarbeitungsmethoden zum Einsatz, die zum Beispiel auf trainierten Modellen bestehen können und/oder die Strukturinformation im Bild nutzen.

Auf Basis dieser Information ist bereits eine Lokalisierung der Pupillen im Raum möglich. Damit ist es implizit möglich einen Skalierungsfaktor von Bildpixel zu einer Längeneinheit an dieser Position zu berechnen.

Die Umgebung des Bildes an der selektierten Pupillenposition kann durch eine Vorverarbeitung modifiziert werden.

Zusätzlich wird ausgenutzt, dass die Pupille das dunkelste Objekt ist. Dies kann beispielsweise dazu genutzt werden, um einen unteren Schwellwert festzulegen, unterhalb dessen die Helligkeit auf einen festen, gleichen Wert gesetzt werden kann. Somit kann die Information, die in der Pupille vorhanden ist, als Artefakt behandelt und somit entfernt werden.

Ein Inkrementieren der entsprechenden Koordinate des Punktes in der Pupille, entlang der längeren der beiden Strecken der entsprechenden Geraden im Sinne der Erfindung meint dabei beispielsweise, dass die beiden Strecken aufaddiert werden können, und dann die entsprechende Koordinate entlang der längeren Strecke, um den Anteil verschoben wird, welcher der kürzeren Strecke zur Länge der Hälfte der aufaddierten Strecken fehlt. Das Inkrementieren kann jedoch auch pixelweise oder um einen festgelegten Wert erfolgen oder auch um eine Prozentzahl der Länge der längeren Strecke.

Durch die erfindungsgemäße Lehre wird der Vorteil erreicht, dass auf komplexe apparative Konstruktionen verzichtet werden kann und der Pupillendurchmesser in natürlichen Gebrauchssituationen vermessen werden kann. Ein weiterer Vorteil ist, dass ein Verfahren für eine genaue Bestimmung des Pupillendurchmessers zur Verfügung gestellt wird, welches für eine helligkeitsabhängige Anpassung eines Brillenglases Glases genutzt werden kann.

Der Gegenstand eines nebengeordneten Anspruches betrifft dabei eine Vorrichtung zum hochgenauen Bestimmen eines Pupillendurchmessers eines Auges. Dabei weist die Vorrichtung auf: Ein Kamerasystem zum räumlichen Erfassen von Objekten. Ein Erfassungsmittel, zum bildgebenden Stereoskopen Erfassen einer Augenregion eines Kopfes, mittels des Kamerasystems, zum Erzeugen eines ersten und eines zweiten stereoskopen Bildes für ein Auge der Augenregion. Ein Transferierungsmittel, zum Transferieren der erfassten Augenregion des Kopfes in einen Farbraum. Ein erstes Ermittlungsmittel zum Ermitteln eines Bereiches innerhalb der erfassten Augenregion, als einen Bereich innerhalb einer Pupille des Auges der erfassten Augenregion. Ein zweites Ermittlungsmittel, zum Ermitteln eines geschlossenen äußeren Randes des Bereiches innerhalb der Pupille. Ein drittes Ermittlungsmittel, zum iterativen Ermitteln eines geschlossenen äußeren Pupillenrandes.

In einer Ausführungsform weist die Vorrichtung weiterhin auf: Ein erstes Bestimmungsmittel, zum Bestimmen eines Punktes in der Pupille. Ein zweites Bestimmungsmittel, zum Bestimmen einer ersten Geraden durch den Punkt. Ein drittes Bestimmungsmittel, zum Bestimmen einer, orthogonal zur ersten Geraden angeordneten, zweiten Geraden durch den Punkt. Ein viertes Ermittlungsmittel, zum iterativen Ermitteln eines Pupillenmittelpunktes.

In einer Ausführungsform weist die Vorrichtung weiterhin auf: Ein erstes Triangulierungsmittel, zum triangulierten Überlagern der Pupillenmittelpunkte der Pupillen des Auges der stereoskopen Bilder. Ein zweites Triangulierungsmittel, zum triangulierten Überlagern der äußeren Ränder der Pupille des Auges der stereoskopen Bilder. Und ein fünftes Ermittlungsmittel, zum Ermitteln des Pupillendurchmessers, basierend auf den beiden triangulierten Überlagerungen. Und dabei ist die Vorrichtung dazu eingerichtet, irgendein erfindungsgemäßes Verfahren auszuführen.

Durch die erfindungsgemäße Lehre wird der Vorteil erreicht, dass eine Vorrichtung bereitgestellt werden kann, mit welcher der Pupillendurchmesser in natürlichen Gebrauchssituationen vermessen werden kann. Ein weiterer Vorteil ist, dass ein Verfahren für eine genaue Bestimmung des Pupillendurchmessers zur Verfügung gestellt wird, welches für eine helligkeitsabhängige Anpassung eines Brillenglases Glases genutzt werden kann.

Der Gegenstand eines weiteren nebengeordneten Anspruches betrifft dabei ein Computerprogrammprodukt für eine Vorrichtung, wobei die Vorrichtung nach irgendeinem erfindungsgemäßen Verfahren betreibbar ist.

Durch die erfindungsgemäße Lehre wird der Vorteil erreicht, dass das Verfahren besonders effizient automatisiert ausgeführt werden kann.

Der Gegenstand eines weiteren nebengeordneten Anspruches betrifft dabei einen Datenträger aufweisend ein erfindungsgemäßes Computerprogrammprodukt.

Durch die erfindungsgemäße Lehre wird der Vorteil erreicht, dass das Verfahren besonders effizient auf die das Verfahren ausführenden Vorrichtungen, Systeme und/oder Kraftfahrzeuge verteilt beziehungsweise vorgehalten werden kann.

Bevor nachfolgend Ausgestaltungen der Erfindung eingehender beschrieben werden, ist zunächst festzuhalten, dass die Erfindung nicht auf die beschriebenen Komponenten oder die beschriebenen Verfahrensschritte beschränkt ist. Weiterhin stellt auch die verwendete Terminologie keine Einschränkung dar, sondern hat lediglich beispielhaften Charakter. Soweit in der Beschreibung und den Ansprüchen der Singular verwendet wird ist dabei jeweils der Plural mit umfasst, soweit der Kontext dies nicht explizit ausschließt. Etwaige Verfahrensschritte können, soweit der Kontext dies nicht explizit ausschließt, automatisiert ausgeführt werden. Entsprechende Verfahrensabschnitte können zu entsprechenden Vorrichtungseigenschaften führen und umgekehrt, so dass, sofern der Kontext dies nicht explizit ausschließt, ein Wechsel eines Verfahrensmerkmales in ein Vorrichtungsmerkmal ermöglicht wird und umgekehrt.

Nachfolgend werden weitere exemplarische Ausgestaltungen des erfindungsgemäßen Verfahrens erläutert.

Entsprechend einer ersten exemplarischen Ausgestaltung weist das Verfahren ferner, vor dem bildgebenden stereoskopen Erfassen der Augenregion des Kopfes, ein Kalibrieren des Kamerasystems zum räumlichen Erfassen von Objekten auf. Dabei erfolgt das Kalibrieren vorzugsweise basierend auf einem vordefinierten Winkel und/oder einem vordefinierten Abstand des Kamerasystems zu einem Bezugsobjekt. Und dabei befindet sich der Kopf der zu erfassenden Augenregion, während der stereoskopen Erfassung an der Position des Bezugsobjektes.

Diese Ausgestaltung weist den Vorteil auf, dass der Pupillendurchmesser damit noch präziser ermittelt werden kann.

Entsprechend einer weiteren exemplarischen Ausgestaltung weist das Verfahren ferner ein Ermitteln eines Auges in der stereoskop-erfassten Augenregion auf, basierend auf einem Abgleich der stereoskop-erfassten Augenregion mit einem Augenmodell.

Diese Ausgestaltung weist den Vorteil auf, dass ein Auge selbständig ermittelt werden kann, wodurch die Pupillendurchmesserbestimmung noch weiter vereinfacht werden kann.

Entsprechend einer weiteren exemplarischen Ausgestaltung weist das Verfahren ferner auf, dass der Farbraum einem Farbraum entspricht, in welchem das Bildkriterium besonders geeignet ermittelbar ist.

Wird das Bild beispielsweise mittels eines RGB-Sensors aufgenommen, kann eine Farbraumtransformation durchgeführt werden. Da die Farbe der Iris unterschiedlich sein kann, ist der RGB-Farbraum nicht gut geeignet, um Helligkeitsunterschiede zu detektieren. Besser geeignet ist beispielsweise der CIELAB-Farbraum bei dem dann zum Beispiels nur der X-Kanal betrachtet wird.

Diese Ausgestaltung weist den Vorteil auf, dass der Rand der Pupille noch präziser ermittelt werden kann.

Entsprechend einer weiteren exemplarischen Ausgestaltung weist das Verfahren ferner auf, dass das Bildkriterium ein Kontrastkriterium aufweist.

Diese Ausgestaltung weist den Vorteil auf, dass der Rand der Pupille noch präziser ermittelt werden kann.

Entsprechend einer weiteren exemplarischen Ausgestaltung weist das Verfahren ferner auf, dass der Farbraum einem CIELAB Farbraum entspricht.

Diese Ausgestaltung weist den Vorteil auf, dass der Rand der Pupille noch präziser ermittelt werden kann.

Entsprechend einer weiteren exemplarischen Ausgestaltung weist das Verfahren ferner auf, dass das Ermitteln des Bereiches innerhalb der erfassten Augenregion, als Bereich innerhalb der Pupille des Auges der erfassten Augenregion, basierend auf einem Pupillenkriterium erfolgt, vorzugsweise basierend auf einem Abgleich mit einem Pupillenmodell erfolgt.

Diese Ausgestaltung weist den Vorteil auf, dass die Pupille einfacher ermittelt werden kann.

Entsprechend einer weiteren exemplarischen Ausgestaltung weist das Verfahren ferner auf: Bestimmen eines weiteren Punktes in der Pupille. Bestimmen einer weiteren ersten Geraden durch den weiteren Punkt. Bestimmen einer weiteren zweiten Geraden durch den weiteren Punkt, wobei die weitere zweite Gerade orthogonal zur weiteren ersten Geraden angeordnet ist. Und dabei weist das iterative Ermitteln des Pupillenmittelpunktes ferner auf: Ermitteln einer weiteren ersten Strecke und einer weiteren zweiten Strecke für jede weitere Gerade, wobei das Ermitteln der jeweiligen Strecke aufweist: Ermitteln eines weiteren ersten Endpunktes der entsprechenden Strecke, wobei der erste weitere Endpunkt einem weiteren Punkt in der Pupille entspricht. Ermitteln eines weiteren zweiten Endpunktes der weiteren entsprechenden Strecke, wobei der weitere zweite Endpunkt einem Kreuzungspunkt der entsprechenden weiteren Geraden mit dem Rand der Pupille entspricht. Und ermitteln der entsprechenden weiteren Strecke, als eine Linie entlang der entsprechenden weiteren Geraden von dem entsprechenden weiteren ersten Endpunkt ausgehend zu dem entsprechenden weiteren zweiten Endpunkt hinführend. Und das iterative Ermitteln des Pupillenmittelpunktes weist dabei ferner auf: Längenmäßiges Vergleichen der beiden weiteren Strecken der weiteren ersten Geraden. Falls die beiden weiteren Strecken der weiteren ersten Geraden nicht gleich lang sind, erfolgt ein Inkrementieren einer weiteren ersten Koordinate des weiteren Punktes in der Pupille, entlang der längeren der beiden weiteren Strecken der weiteren ersten Geraden. Falls die beiden weiteren Strecken der weiteren zweiten Geraden nicht gleich lang sind, erfolgt ein Inkrementieren einer weiteren zweiten Koordinate des weiteren Punktes in der Pupille, entlang der längeren der beiden weiteren Strecken der weiteren zweiten Geraden. Und falls die beiden weiteren Strecken der weiteren ersten Geraden gleichlang sind und die beiden weiteren Strecken der zweiten weiteren Geraden gleichlang sind, erfolgt ein Festlegen des weiteren Punktes in der Pupille als einen weiteren Mittelpunkt der Pupille des Auges.

Dies kann dann sinnvoll werden, wenn beispielsweise mittels eines Objekterkennungsverfahrens die Flächigkeit der Pupille als nicht kreisrund, sondern elliptisch erkannt wurde und somit zwei Punkte als Mittelpunkte fungieren. Um diese beiden Mittelpunkte akkurat bestimmen zu können, kann es mathematisch komplexerer Vorgänge bedürfen, wie sie zur Brennpunktbestimmung einer Ellipse notwendig sind. Dabei liegen die Verbindungsgeraden von einem Punkt auf der Ellipse zu den zwei Brennpunkten spiegelbildlich zur Normalen zur Ellipse in diesem Punkt.

Diese Ausgestaltung weist den Vorteil auf, dass der Pupillendurchmesser auch bei stark ellipsenförmigen Pupillenabbildungen bzw. stark ellipsenförmigen Pupillen mit hoher Präzision ermittelbar ist.

Entsprechend einer weiteren exemplarischen Ausgestaltung weist das Verfahren ferner auf: Trianguliertes Überlagern der weiteren beiden Mittelpunkte der Pupillen des Auges der beiden stereoskopen Bilder. Trianguliertes Überlagern der beiden äußeren Ränder der Pupille des Auges der beiden stereoskopen Bilder. Und ermitteln des weiteren Pupillendurchmessers, basierend auf den beiden triangulierten Überlagerungen.

Diese Ausgestaltung weist den Vorteil auf, dass der Pupillendurchmesser noch präziser ermittelt werden kann.

Entsprechend einer weiteren exemplarischen Ausgestaltung weist das Verfahren ferner ein Ermitteln eines Größen-Maßstabes auf, basierend auf einer Entfernung des Kamerasystems zu der stereoskop-erfassten Augenregion des Kopfes und eines Pixelkriteriums des Kamerasystems. Und dabei erfolgt das Ermitteln des jeweiligen Pupillendurchmessers basierend auf dem ermittelten Größen-Maßstab.

Diese Ausgestaltung weist den Vorteil auf, dass der Pupillendurchmesser noch präziser ermittelt werden kann.

Entsprechend einer weiteren exemplarischen Ausgestaltung weist das Verfahren ferner auf, dass das Pixelkriterium des Kamerasystems eine Pixelgröße beziehungsweise Pixelbreite und eine Pixelanzahl aufweist.

Diese Ausgestaltung weist den Vorteil auf, dass der Pupillendurchmesser noch präziser ermittelt werden kann.

Entsprechend einer weiteren exemplarischen Ausgestaltung weist das Verfahren ferner, vor dem Ermitteln des Bereiches innerhalb der erfassten Augenregion, als den Bereich innerhalb der Pupille des Auges der erfassten Augenregion, auf: Untersuchen der erfassten Augenregion auf Reflektionen. Und falls die erfasste Augenregion mindestens eine Reflexion aufweist, weist das Verfahren ferner ein Entfernen der auftretenden Reflexion mittels eines Nachbarschaftskriteriums auf, derart, dass eine Bildinformation im einem Bereich in welchem die Reflexion auftritt, mit entsprechenden Bildinformationen der auf dem Nachbarschaftskriterium basierenden entsprechenden Nachbarpixel ersetzt werden.

Relevante Schritte sind hier die Detektion von Beleuchtungsreflexen in denen eine hohe Helligkeit auftritt beziehungsweise das Bild übersättigt ist, innerhalb der Pupille und insbesondere am Rand der Pupille.

Diese Bereiche werden durch Bildinformation aus der Umgebung überschrieben, sogenanntes ,Inpainting', beispielsweise mittels eines next/closest neighbour' Verfahrens.

Diese Ausgestaltung weist den Vorteil auf, dass der Rand der Pupille noch genauer ermittelbar ist.

Entsprechend einer weiteren exemplarischen Ausgestaltung weist das Verfahren ferner auf: Durchführen des Verfahrens für ein erstes Auge der stereoskop-erfassten Augenregion. Und Durchführen des Verfahrens für ein zweites Auge der stereoskop-erfassten Augenregion.

Diese Ausgestaltung weist ferner den Vorteil auf, dass für beide Augen in einem Schritt die Pupillendurchmesser präzise bestimmt werden können. Dadurch kann die Pupillendurchmesserbestimmung für beide Augen mit derselben Aufnahme durchgeführt werden.

Ferner kann auch ausgenutzt werden, dass die Pupille keine Struktur aufweist, die Iris typischerweise jedoch Struktur aufweist. Durch die Berechnung der lokalen Entropie des Bildes kann dieser Unterschied ebenfalls ausgenutzt werden.

Ferner kann ein entsprechend vorverarbeitetes Bild in Polarkoordinaten transformiert werden, mit einem selektierten Pupillenmittelpunkt als Ursprung. Dadurch erscheint ein kreisrunder Dunkel-hell-Übergang als Linie. Eine Ellipse würde als eine Sinus/Kosinus-Kurve abgebildet. In diesem transformierten Bild kann nun nach einer Dunkel-hell Kante gesucht werden. Dies kann mit üblichen Kanten-Filtern, wie beispielsweise Canny, Sobel und dergleichen erfolgen. Die Koordinate der bestimmten Kante entspricht dann dem mittleren Radius der Pupille in Pixel.

Die Umrechnung von Pixel in eine Längeneinheit kann mithilfe eines Skalierungsfaktors vorgenommen werden, der sich aus der Triangulation ergibt.

Eine noch genauere Mittenbestimmung, Pupillenform kann folgendermaßen erfolgen:
Anstatt die Dunkel-hell Kante über den gesamten Winkelbereich zu detektieren kann dies auch segmentweise erfolgen. Bei einer Verwendung von beispielsweise vier Segmenten ist es möglich eine Abweichung des selektierten Pupillenmittelpunktes vom tatsächlichen Mittelpunkt zu ermitteln, wenn gegenüberliegende Segmente Differenzen in den ermittelten Radien aufweisen. Es ist dann möglich diese Differenz zum Neupositionieren des Pupillenmittelpunktes zu nutzen. Eine Überprüfung ist leicht durch nochmalige Anwendung des Verfahrens möglich. Vorzugsweise würde man eine kleinere Anpassung des Mittelpunktes als die Hälfte der Differenz vornehmen und häufiger überprüfen, bis die Differenz unter einen festgelegten Schwellwert gesunken ist und somit der genaue Pupillenmittelpunkt ermittelt ist.

Bei mehr als vier Segmenten ist zudem eine Detektion komplexerer Formen wie beispielsweise einer Ellipse möglich beziehungsweise bei noch mehr Segmenten eine andere Parametrierung der Pupillenform, wie beispielsweise als N-Ellipse oder als Spline und dergleichen.

Die Ellipse wird im Bild als Form einer idealen, kreisförmigen Pupille erwartet, da die Pupillenebene im Allgemeinen nicht parallel zur Bildebene der aufnehmenden Kamera steht beziehungsweise nicht auf beide Bildebenen parallel stehen kann. Deren Detektion bedeutet also eine Verbesserung der Genauigkeit hinsichtlich des Pupillendurchmessers.

Ferner ist ein Vergleich zwischen den beiden Aufnahmen des Stereokamerasystems von Vorteil, da ein Abgleich zwischen diesen vorgenommen werden kann, um die Plausibilität der gefundenen Pupillenform zu überprüfen.

Aus den Radiensegmenten können Punkte auf dem Übergang Pupille zu Iris in beiden Aufnahmen ausgewählt werden. Diese können über Triangulation zu einem Punkt im Raum kombiniert werden. Werden drei oder mehr Segmente verwendet, lässt sich daraus die Pupillenebene bestimmen. Die optische Achse des Auges und die Blickrichtung können als senkrecht auf diese Ebene stehend angenommen werden beziehungsweise mit einem bekannten, festen Winkel. Somit ist daraus die Bestimmung der Blickrichtung möglich.

Vorzugsweise können deutlich mehr als drei Punkte für die Ebenenbestimmung verwendet werden. Es bieten sich hier 4, 6, 8, 12, 16, 24, 32 oder 64 Segmente an.

Zur Überprüfung der 3D-Kontur des Übergangs Pupille Iris beziehungsweise der Pupillenform kann für die zu triangulierenden Punkte die Sampson Entfernung, also eine Näherung erster Ordnung für den geometrischen Fehler, berechnet werden. Weist dieser für ein Punktepaar einen ungewöhnlich hohen Wert auf, so ist hier eher von einer Fehldetektion auszugehen anstatt von einer Abweichung von der Kreis-/Ellipsenform. Für dieses Punktepaar kann dann entweder mit modifizierten Parametern erneut versucht werden eine Detektion durchzuführen oder das Punktepaar wird verworfen.

Aus der Blickrichtung eines rechten und eines linken Auges kann ein Fixierpunkt ermittelt werden. Dies kann zum Beispiel der Punkt sein, an dem die beiden Geraden, welche die Blickrichtung beschreiben, den kleinsten Abstand zueinander haben. Der Fixierpunkt ist im Weltkoordinatensystem, in dem auch die anderen Punkte bekannt sind, bekannt.

Damit wäre auch die Bestimmung einer Leseentfernung ohne entsprechende Vorrichtungen möglich.

Die Erfindung erlaubt es somit, mittels einer Vorrichtung, die ein Stereokamerasystem aufweist, simultan Aufnahmen in normalen Gebrauchs- und Umbgebungssituationen eines Probanden mit den beiden Kameras vorzunehmen, die den Augenbereich des Probanden umfassen. Durch eine vorbekannte Kalibrierung der Kameras können aus diesen Aufnahmen 3D Positionsdaten von ausgewählten Objektpunkten sehr präzise ermittelt werden.

In der vorliegenden Erfindung wird in den Einzelbildern zunächst ein Pupillenrand ermittelt. Dies kann schrittweise, beispielsweise zunächst Gesicht beziehungsweise Augenbereich, dann Auge, dann Pupillenmittelpunkt, dann Pupillenrand, und dabei semi-automatisch oder vollautomatisch erfolgen. Der Pupillenrand bezeichnet hier die weitestgehend runde Form die den Übergang zwischen Pupille und Iris eines Auges beschreibt. Durch die perspektivische Aufnahme würde eine ideal kreisrunde Pupille als Ellipse dargestellt. Weitere Abweichungen von einer Kreisform haben weitere perspektivische Verzerrungen zur Folge. Nach erfolgreicher Bestimmung der Pupillenränder in beiden Bildern, das heißt jeweils in 2D, wird die 3D Position und Form des Pupillenrandes bestimmt.

Zumindest Näherungsweise kann davon ausgegangen werden, dass die Normale auf die Fläche die durch den Pupillenrand beschrieben wird der Blickrichtung entspricht. Somit kann die Blickrichtung ermittelt werden.

Erfolgt die Ermittlung der Blickrichtung für beide Augen, kann ein möglicher Kreuzungspunkt der beiden Blickrichtungen bestimmt werden. Im Allgemeinen werden sich die Blickrichtungen der beiden Augen nicht exakt schneiden. Der Kreuzungspunkt kann beispielsweise als der Punkt bestimmt werden, der den kleinsten Abstand der Blickrichtungen aufweist oder als der Kreuzungspunkt in einer Projektion entlang einer geraden die mittig durch die beiden Pupillen verläuft, das heißt, weitestgehend senkrecht ist, die Projektion somit in einer horizontalen Ebene erfolgt. Der Abstand des Kreuzungspunktes zu der Pupillenposition kann somit zur Bestimmung eines Objektabstandes verwendet werden.

Die 3D Position und Form des Pupillenrandes kann genutzt werden um daraus einen genauen Pupillendurchmesser oder Radius zu bestimmen. Dabei wird die Form in Ihrer 2D Projektion an einen Kreis angenähert, dessen Radius dann als Pupillenradius verwendet wird. Sowohl für die Projektion in 2D als auch für die Kreisanpassung sind in der Literatur vielfältige Ausgleichsverfahren beschrieben. Möglich ist beispielsweise die Anpassung unter Verwendung einer Zielfunktion die minimiert werden soll. Die Zielfunktion kann beispielsweise die Summe der Abstandsquadrate zwischen Messpunkten und genäherten Punkten beschreiben.

Da der Objektabstand ermittelt werden kann ohne dass ein Fixationsobjekt nötig ist, können beliebige Sehsituationen vermessen werden. Hilfreich ist dies beispielsweise zur Ermittlung von Distanzen die bei der Verwendung von bestimmten Geräten nutzerspezifisch eingenommen werden, wie beispielsweise Monitore am Arbeitsplatz, handgehaltene Ein-/Ausgabegeräte wie Smartphones, Tablets, Prüfgeräte mit Displays, Anzeigeinstrumente in einem Cockpit und dergleichen oder auch andere weitgehend statische aktivitätsspezifische Abstände, wie beispielsweise Bücher lesen, Golf spielen, Handarbeiten, Radfahren und dergleichen. Dynamischere Situationen können über eine Vielzahl von Messungen statistisch abgebildet werden.

Unter Umständen können nicht alle individuellen Parameter bei solch einer einzelnen Messung ermittelt werden. Dazu zählt beispielsweise die Vorneigung, die abhängig von der Lage der optischen Achsen der aufnehmenden Kameras ist. Diese Daten können jedoch durch einen Vergleich mit einem Datensatz unter entsprechend definierten Bedingungen vervollständigt werden. Beispielsweise könnte eine erste Messung mit horizontaler erster optischer Achse durchgeführt werden und eine zweite Messung in einer Anwendungssituation, zum Beispiel wie bei einer Handarbeit am Tisch mit gebeugtem Kopf. Über die in der zweiten Messung ermittelbaren Zentrierdaten und Objektabstände ist zusammen mit der ersten Messung ein vollständiger Datensatz mit zwei Objektabstandsinformationen bezogen auf einen Punkt in der Scheibenebene erhältlich. Alternativ ist auch eine Korrektur des Winkels der ersten optischen Achse möglich wenn dieser durch eine zusätzliche Vorrichtung zum Aufnahmezeitpunkt ermittelt wird.

Alternativ kann die Ermittlung des Kreuzungspunktes der Blickrichtungen auch dazu genutzt werden, die Einhaltung einer vorgegebenen Sehaufgabe mit Fixation eines definierten Objektes zu überprüfen. So kann bei Verwendung der Nasenwurzelmethode der Abstand der Nasenwurzel im Spiegelbild ermittelt werden und mit dem Abstand durch die Kreuzungspunkte verglichen werden. Bei zu großen Abweichungen kann die Messung für ungültig erklärt werden oder dem Bediener ein Hinweis gegeben werden, worauf dieser die Fixation des Probanden überprüfen und gegebenenfalls korrigieren kann.

Mit dem hier beschriebenen Verfahren kann somit auf komplexe apparative Vorrichtungen verzichtet werden, da der Abstand zum Fixierobjekt ermittelt werden kann und somit keine Kooperation des Kunden erforderlich ist.

### Kurzdarstellung der Figuren

Die Erfindung wird nachfolgend eingehender an Hand der Figuren erläutert werden. In diesen zeigen:
Fig. 1 eine schematische Darstellung eines vorgeschlagenen Verfahrens gemäß einer beispielhaften Ausgestaltung der Erfindung;
Fig. 2 eine schematische Darstellung eines vorgeschlagenen Verfahrens gemäß einer weiteren beispielhaften Ausgestaltung der Erfindung;
Fig. 3 eine schematische Darstellung eines vorgeschlagenen Verfahrens gemäß einer weiteren beispielhaften Ausgestaltung der Erfindung;
Fig. 4 eine schematische Darstellung eines vorgeschlagenen Verfahrens gemäß einer weiteren beispielhaften Ausgestaltung der Erfindung;
Fig. 5 eine schematische Darstellung eines vorgeschlagenen Verfahrens gemäß einer weiteren beispielhaften Ausgestaltung der Erfindung;
Fig. 6 eine schematische Darstellung eines vorgeschlagenen Verfahrens gemäß einer weiteren beispielhaften Ausgestaltung der Erfindung;
Fig. 7 eine schematische Darstellung eines vorgeschlagenen Verfahrens gemäß einer weiteren beispielhaften Ausgestaltung der Erfindung;
Fig. 8 eine schematische Darstellung eines vorgeschlagenen Verfahrens gemäß einer weiteren beispielhaften Ausgestaltung der Erfindung;
Fig. 9 eine schematische Darstellung eines vorgeschlagenen Verfahrens gemäß einer weiteren beispielhaften Ausgestaltung der Erfindung;
Fig. 10 eine schematische Darstellung eines vorgeschlagenen Verfahrens gemäß einer weiteren beispielhaften Ausgestaltung der Erfindung; und
Fig. 11 eine schematische Darstellung einer vorgeschlagenen Vorrichtung gemäß einer weiteren beispielhaften Ausgestaltung der Erfindung.

### Ausführliche Beschreibung

Nachfolgend wird die Erfindung eingehender (unter Bezugnahme auf die Figuren) dargestellt werden. Dabei ist anzumerken, dass unterschiedliche Aspekte beschrieben werden, die jeweils einzeln oder in Kombination zum Einsatz kommen können. D.h. jeglicher Aspekt kann mit unterschiedlichen Ausführungsformen der Erfindung verwendet werden, soweit nicht explizit als reine Alternative dargestellt.

Weiterhin wird nachfolgend der Einfachheit halber in aller Regel immer nur auf eine Entität Bezug genommen werden. Soweit nicht explizit vermerkt, kann die Erfindung aber auch jeweils mehrere der betroffenen Entitäten aufweisen. Insofern ist die Verwendung der Wörter "ein", "eine" und "eines" nur als Hinweis darauf zu verstehen, dass in einer einfachen Ausführungsform zumindest eine Entität verwendet wird. Soweit nachfolgend Verfahren beschrieben werden, sind die einzelnen Schritte eines Verfahrens in beliebiger Reihenfolge anordenbar und/oder kombinierbar, soweit sich durch den Zusammenhang nicht explizit etwas Abweichendes ergibt. Weiterhin sind die Verfahren - soweit nicht ausdrücklich anderweitig gekennzeichnet - untereinander kombinierbar.

Angaben mit Zahlenwerten sind in aller Regel nicht als exakte Werte zu verstehen, sondern beinhalten auch eine Toleranz von +/- 1% bis zu +/- 10 %.

Bezugnahme auf Standards oder Spezifikationen oder Normen sind als Bezugnahme auf Standards bzw. Spezifikationen bzw. Normen, die zum Zeitpunkt der Anmeldung und/oder - soweit eine Priorität beansprucht wird - zum Zeitpunkt der Prioritätsanmeldung gelten / galten, zu verstehen. Hiermit ist jedoch kein genereller Ausschluss der Anwendbarkeit auf nachfolgende oder ersetzende Standards oder Spezifikationen oder Normen zu verstehen.

"Benachbart" schließt im Nachfolgenden explizit eine unmittelbare Nachbarschaftsbeziehung ein ohne jedoch hierauf beschränkt zu sein. "Zwischen" schließt im Nachfolgenden explizit eine Lage ein, in der das zwischenliegende Teil eine unmittelbare Nachbarschaft zu den umgebenden Teilen aufweist.

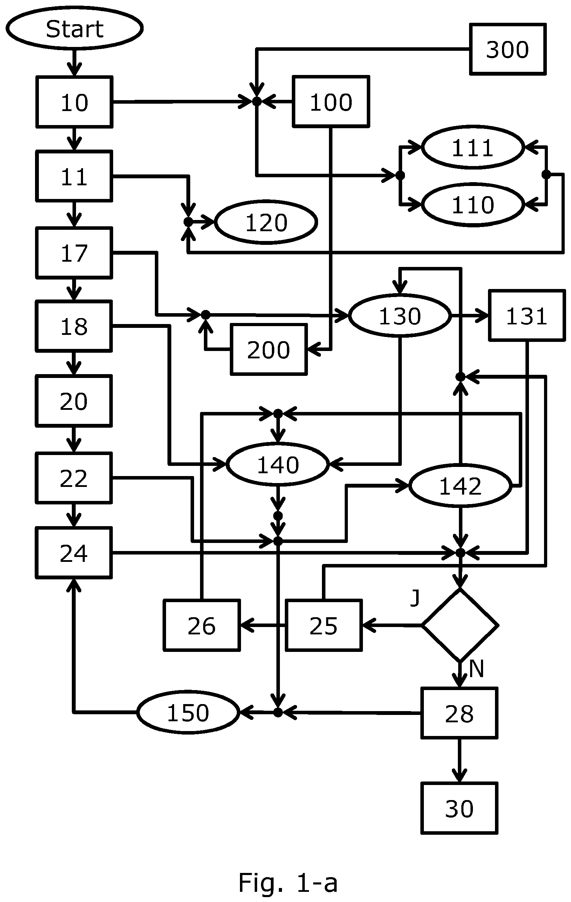

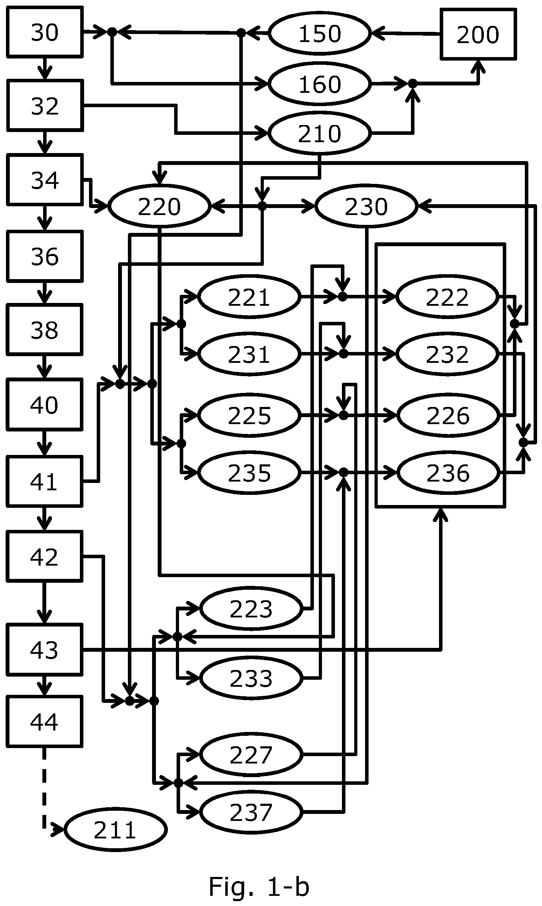

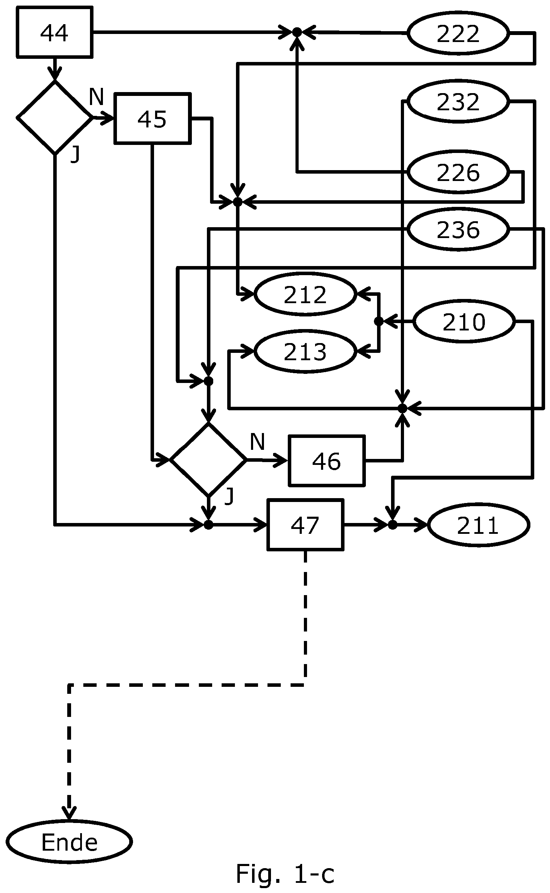

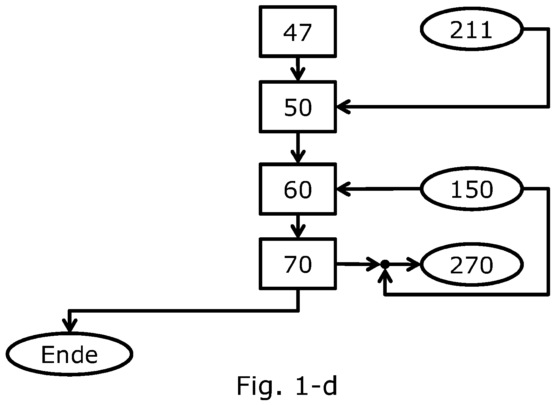

Fig. 1 zeigt eine schematische Darstellung eines vorgeschlagenen Verfahrens gemäß einer beispielhaften Ausgestaltung der Erfindung.

Dabei zeigt Fig. 1 eine schematische Darstellung eines Verfahrens zum hochgenauen Bestimmen eines Pupillendurchmessers 270 eines Auges, mittels eines Kamerasystems 300 zum räumlichen Erfassen von Objekten. Dabei weist das Verfahren auf:

### (Fig. 1-a):

Bildgebendes stereoskopes Erfassen 10 einer Augenregion 100 eines Kopfes, mittels des Kamerasystems 300, wobei sich für ein Auge der Augenregion 100 ein erstes und ein zweites stereoskopes Bild 110, 111 ergeben. Und für ein jedes der beiden stereoskopen Bilder 110, 111 weist das Verfahren ferner auf: Transferieren 11 der erfassten Augenregion 100 des Kopfes in einen Farbraum 120, und weiterführen des Verfahrens in diesem Farbraum 120. Ermitteln 17 eines Bereiches 130 innerhalb der erfassten Augenregion 100, als einen Bereich 130 innerhalb einer Pupille 200 des Auges der erfassten Augenregion 100, wobei der ermittelte Bereich 130 innerhalb der erfassten Augenregion 100 ein Bildkriterium 131 aufweist. Dabei stellt das Bildkriterium 131 ein weitgehend homogenes Bildkriterium dar. Ermitteln 18 eines geschlossenen äußeren Randes 140 des Bereiches 130 innerhalb der Pupille 200. Iteratives Ermitteln 20 eines geschlossenen äußeren Pupillenrandes 150, das iterative Ermitteln 20 des äußeren Pupillenrandes 150 aufweisend: Den äußeren Rand 140 umlaufendes Ermitteln 22 eines weiteren Bereiches 142 außerhalb des Randes 140, entlang des Randes 140, wobei der weitere Bereich 142 an den geschlossenen äußeren Rand 140 des Bereiches 130 innerhalb der Pupille 200 angrenzt. Iteratives Untersuchen 24 des weiteren Bereiches 142 daraufhin, ob der weitere Bereich 142 das Bildkriterium 131 aufweist. Und falls zumindest ein Teil des weiteren Bereiches 142 das Bildkriterium 131 aufweist: Integrieren 25 desjenigen Teiles des weiteren Bereiches 142, welcher das Bildkriterium 131 aufweist, in den Bereich 130 innerhalb der Pupille 200 des Auges. Ausweiten 26 des geschlossenen äußeren Randes 140 des Bereiches 130 innerhalb der Pupille 200 auf den integrierten Teil des weiteren Bereiches 142. Und falls der weitere Bereich 132 das Bildkriterium 131 vollständig nicht aufweist: Festlegen 28 des geschlossenen äußeren Randes 140 des Bereiches 130 innerhalb der Pupille 200 als den äußeren Rand 150 der Pupille 200 des Auges.

### Fig. 1-b:

Und ermitteln 30 eines dritten Bereiches 160 als Pupille 200 des Auges, wobei der dritte Bereich 160 derjenige Bereich ist, der vollständig vom äußeren Rand 150 der Pupille 200 eingeschlossen ist.

In Ausführungsformen der Erfindung kann dann wie folgt verfahren werden: Bestimmen 32 eines Punktes 210 in der Pupille 200. Bestimmen 34 einer ersten Geraden 220 durch den Punkt 210. Bestimmen 36 einer zweiten Geraden 230 durch den Punkt 200, wobei die zweite Gerade 230 orthogonal zur ersten Geraden 220 angeordnet ist. Und iteratives Ermitteln 38 eines Pupillenmittelpunktes 211, wobei das iterative Ermitteln 38 des Pupillenmittelpunktes 211 aufweist: Ermitteln 40 einer ersten Strecke 222, 232 und einer zweiten Strecke 226, 236 für jede Gerade 220, 230, wobei das Ermitteln 40 der jeweiligen Strecke 222, 232, 226, 236 aufweist: Ermitteln 41 eines ersten Endpunktes 221, 231, 225, 235 der entsprechenden Strecke 222, 232, 226, 236, wobei der erste Endpunkt 221, 231, 225, 235 dem Punkt 210 in der Pupille 200 entspricht. Ermitteln 42 eines zweiten Endpunktes 223, 233, 227, 237 der entsprechenden Strecke 222, 232, 226, 236, wobei der zweite Endpunkt 223, 233, 227, 237 einem Kreuzungspunkt der entsprechenden Geraden 220, 230 mit dem Rand der Pupille 150 entspricht. Und ermitteln 43 der entsprechenden Strecke 222, 232, 226, 236, als eine Linie entlang der entsprechenden Geraden 220, 230 von dem entsprechenden ersten Endpunkt 221, 231, 225, 235 ausgehend zu dem entsprechenden zweiten Endpunkt 223, 233, 227, 237 hinführend.

### Fig. 1-c:

Iteratives längenmäßiges Vergleichen 44 der beiden Strecken 222, 226 der ersten Geraden 220. Und falls die beiden Strecken 222, 226 der ersten Geraden 220 nicht gleich lang sind: Inkrementieren 45 einer ersten Koordinaten 212 des Punktes 210 in der Pupille 200, entlang der längeren der beiden Strecken 222, 226 der ersten Geraden 220. Und falls die beiden Strecken 232, 236 der zweiten Geraden 230 nicht gleich lang sind: Inkrementieren 46 einer zweiten Koordinaten 213 des Punktes in der Pupille 200, entlang der längeren der beiden Strecken 232, 236 der zweiten Geraden 230. Und falls die beiden Strecken 222, 226 der ersten Geraden 220 gleichlang sind und die beiden Strecken 232, 236 der zweiten Geraden 230 gleichlang sind: Festlegen 47 des Punktes 210 in der Pupille 200 als den Pupillenmittelpunkt 211 der Pupille 200 des Auges.

### (Fig. 1-d):

In Ausführungsformen der Erfindung kann dann wie folgt verfahren werden: Trianguliertes Überlagern 50 der beiden Mittelpunkte 211 der Pupillen 200 des Auges der beiden stereoskopen Bilder 110, 111. Trianguliertes Überlagern 60 der beiden äußeren Ränder 150 der Pupille 200 des Auges der beiden stereoskopen Bilder 110, 111. Und ermitteln 70 des Pupillendurchmessers 270, basierend auf den beiden triangulierten Überlagerungen 50, 60.

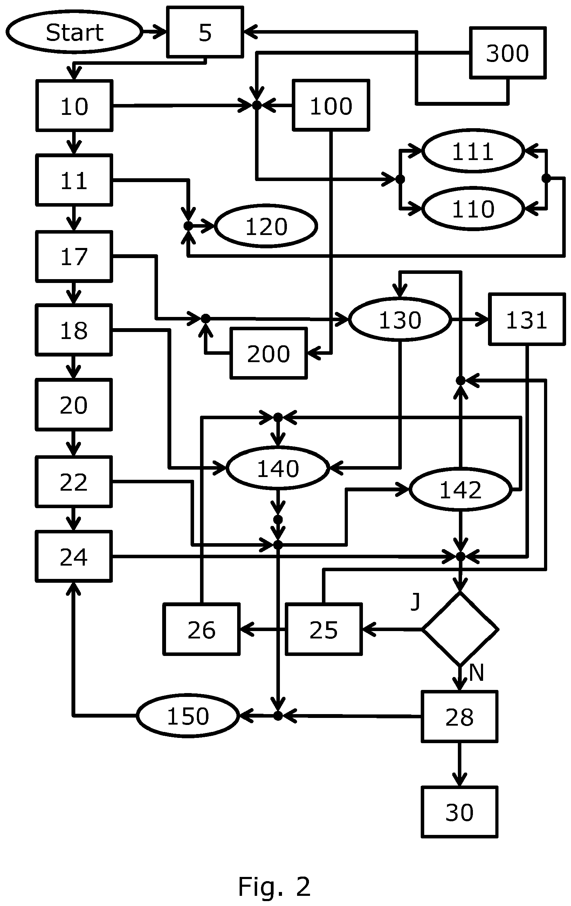

Fig. 2 zeigt eine schematische Darstellung eines vorgeschlagenen Verfahrens gemäß einer weiteren beispielhaften Ausgestaltung der Erfindung.

Dabei zeigt Fig. 2 eine schematische Darstellung eines bezüglich der Fig. 1 beziehungsweise Fig. 1-a weiterentwickelten Verfahrens. Das zuvor für Fig. 1 Gesagte, gilt daher auch für Fig. 2 fort.

Dabei zeigt Fig. 2 eine schematische Darstellung eines vorgeschlagenen Verfahrens, wobei das Verfahren zusätzlich zu dem Verfahrensausschnitt der Fig. 1-a aufweist: Kalibrieren 5 des Kamerasystems 300 zum räumlichen Erfassen von Objekten, vor dem bildgebenden stereoskopen Erfassen 10 der Augenregion 100 des Kopfes. Dabei erfolgt das Kalibrieren 5 vorzugsweise basierend auf einem vordefinierten Winkel und/oder einem vordefinierten Abstand des Kamerasystems 300 zu einem Bezugsobjekt. Und dabei befindet sich der Kopf der zu erfassenden Augenregion 100, während der stereoskopen Erfassung 10 an der Position des Bezugsobjektes.

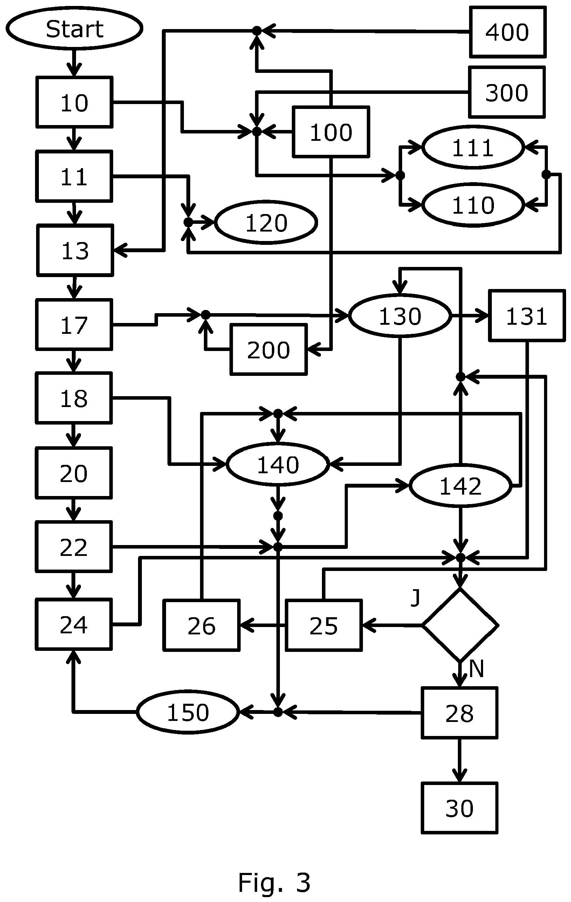

Fig. 3 zeigt eine schematische Darstellung eines vorgeschlagenen Verfahrens gemäß einer weiteren beispielhaften Ausgestaltung der Erfindung.

Dabei zeigt Fig. 3 eine schematische Darstellung eines bezüglich der Fig. 1 und Fig. 2 weiterentwickelten Verfahrens. Das zuvor für Fig. 1 und 2 Gesagte, gilt daher auch für Fig. 3 fort.

Dabei zeigt Fig. 3 eine schematische Darstellung eines vorgeschlagenen Verfahrens, wobei das Verfahren ferner, ein Ermitteln 13 eines Auges in der stereoskop-erfassten 10 Augenregion 100 aufweist, basierend auf einem Abgleich der stereoskop-erfassten 10 Augenregion 100 mit einem Augenmodell 400.

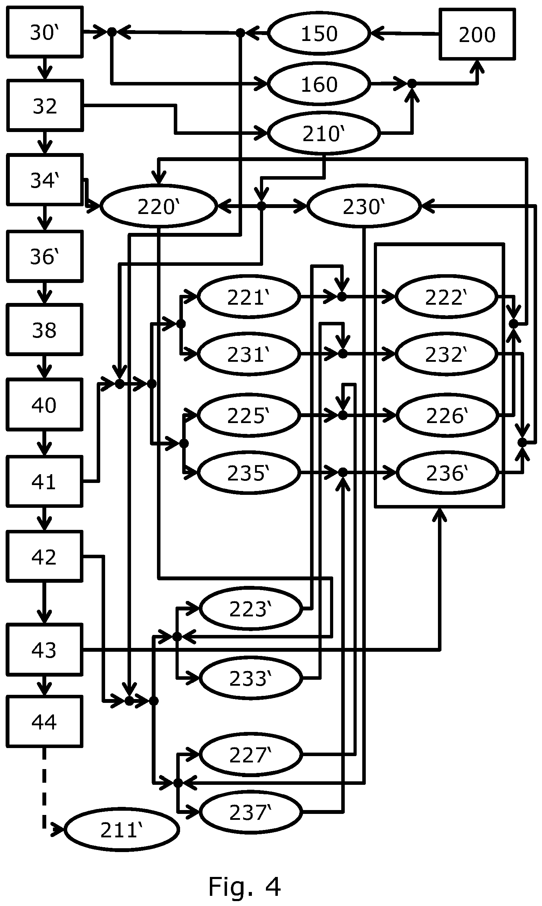

Fig. 4 zeigt eine schematische Darstellung eines vorgeschlagenen Verfahrens gemäß einer weiteren beispielhaften Ausgestaltung der Erfindung.

Dabei zeigt Fig. 4 eine schematische Darstellung eines bezüglich der Fig. 1 bis 3 weiterentwickelten Verfahrens. Das zuvor für Fig. 1 bis 3 Gesagte, gilt daher auch für Fig. 4 fort.

Dabei zeigt Fig. 4 eine schematische Darstellung eines vorgeschlagenen Verfahrens, wobei das Verfahren ferner aufweist: Bestimmen 32' eines weiteren Punktes 210' in der Pupille 200. Bestimmen 34' einer weiteren ersten Geraden 220' durch den weiteren Punkt 210'. Bestimmen 36' einer weiteren zweiten Geraden 230' durch den weiteren Punkt 210', wobei die weitere zweite Gerade 230' orthogonal zur weiteren ersten Geraden 220' angeordnet ist. Und dabei erfolgt das iterative Ermitteln 38 des Pupillenmittelpunktes 211 ferner respektive entsprechend mit dem weiteren Punkt 210', der weiteren ersten Geraden 220' und der weiteren zweiten Geraden 230'. Und als Resultat erfolgt ein entsprechendes Festlegen des weiteren Punktes 210' in der Pupille 200, als einen weiteren Mittelpunkt 211' der Pupille 200 des Auges.

Die gestrichelten Bezugszeichen kennzeichnen dabei jeweils zugehörige Bezüge des weiteren Punktes 210' in Analogie zu den zugehörigen Bezügen des Punktes 210.

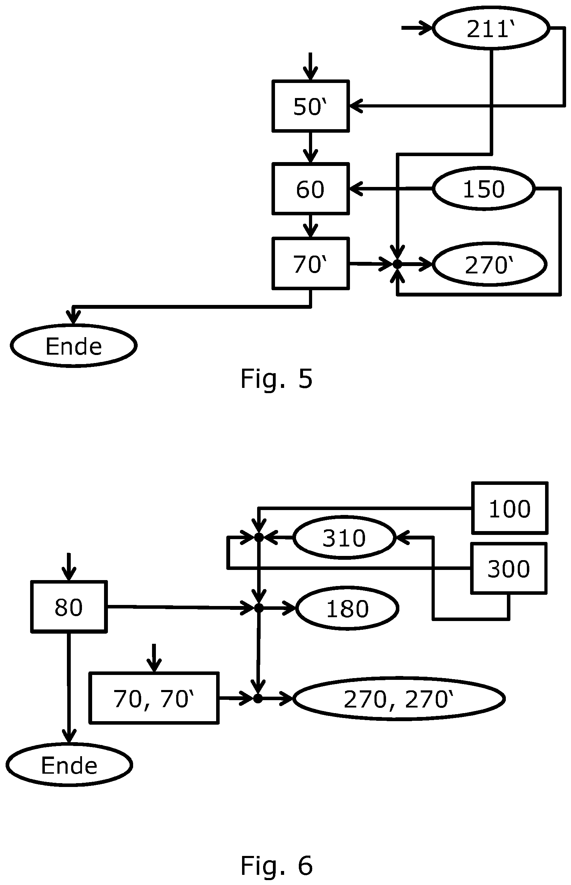

Fig. 5 zeigt eine schematische Darstellung eines vorgeschlagenen Verfahrens gemäß einer weiteren beispielhaften Ausgestaltung der Erfindung.

Dabei zeigt Fig. 5 eine schematische Darstellung eines bezüglich der Fig. 4 weiterentwickelten Verfahrens. Das zuvor für Fig. 4 Gesagte, gilt daher auch für Fig. 5 fort.

Dabei zeigt Fig. 5 eine schematische Darstellung eines vorgeschlagenen Verfahrens, wobei das Verfahren ferner aufweist: Trianguliertes 50' Überlagern der weiteren beiden Mittelpunkte 211' der Pupillen 200 des Auges der beiden stereoskopen Bilder 110, 111. Und Ermitteln 70' des weiteren Pupillendurchmessers 270', basierend auf den beiden triangulierten Überlagerungen 50', 60.

Fig. 6 zeigt eine schematische Darstellung eines vorgeschlagenen Verfahrens gemäß einer weiteren beispielhaften Ausgestaltung der Erfindung.

Dabei zeigt Fig. 6 eine schematische Darstellung eines bezüglich der Fig. 1 bis 5 weiterentwickelten Verfahrens. Das zuvor für Fig. 1 bis 5 Gesagte, gilt daher auch für Fig. 6 fort.

Dabei zeigt Fig. 6 eine schematische Darstellung eines vorgeschlagenen Verfahrens, wobei das Verfahren ferner ein Ermitteln 80 eines Größen-Maßstabes 180 aufweist, basierend auf einer Entfernung des Kamerasystems 300 zu der stereoskop-erfassten Augenregion des Kopfes und eines Pixelkriteriums 310 des Kamerasystems 300. Und dabei erfolgt das Ermitteln 70, 70' des jeweiligen Pupillendurchmessers 270, 270' basierend auf dem ermittelten Größen-Maßstab 180.

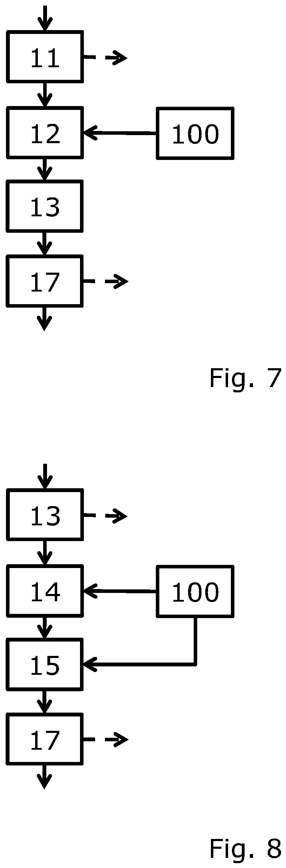

Fig. 7 zeigt eine schematische Darstellung eines vorgeschlagenen Verfahrens gemäß einer weiteren beispielhaften Ausgestaltung der Erfindung.

Dabei zeigt Fig. 7 eine schematische Darstellung eines bezüglich der Fig. 1 bis 6 weiterentwickelten Verfahrens. Das zuvor für Fig. 1 bis 6 Gesagte, gilt daher auch für Fig. 7 fort.

Dabei zeigt Fig. 7 eine schematische Darstellung eines vorgeschlagenen Verfahrens, wobei das Verfahren ferner, vor dem Ermitteln 17 des Bereiches 130 innerhalb der erfassten Augenregion 100, als den Bereich 130 innerhalb der Pupille 200 des Auges der erfassten Augenregion 100, aufweist: Untersuchen 12 der erfassten Augenregion 100 auf Reflektionen. Und falls die erfasste Augenregion 100 mindestens eine Reflexion aufweist: Entfernen 13 der auftretenden Reflexion beziehungsweise Reflexionen mittels eines Nachbarschaftskriteriums, derart, dass eine Bildinformation im einem Bereich in welchem die Reflexion auftritt, mit entsprechenden Bildinformationen der auf dem Nachbarschaftskriterium basierenden entsprechenden Nachbarpixel ersetzt werden.

Fig. 8 zeigt eine schematische Darstellung eines vorgeschlagenen Verfahrens gemäß einer weiteren beispielhaften Ausgestaltung der Erfindung.

Dabei zeigt Fig. 8 eine schematische Darstellung eines vorgeschlagenen Verfahrens, wobei das Verfahren ferner ein Durchführen des Verfahrens 14 für ein erstes Auge der stereoskop-erfassten Augenregion 100 und ein Durchführen des Verfahrens 15 für ein zweites Auge der stereoskop-erfassten Augenregion 100 aufweist.

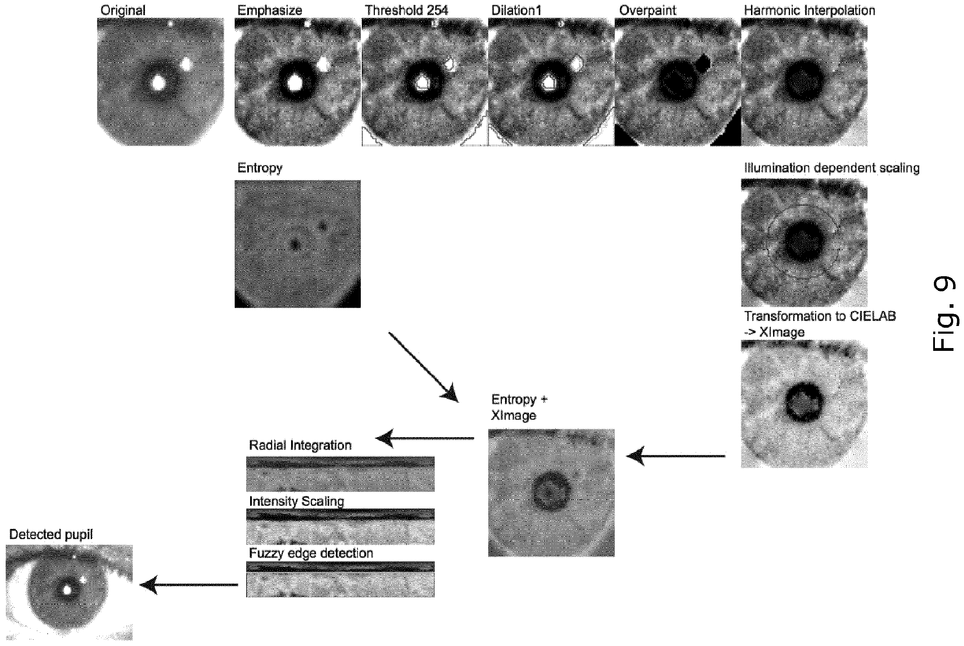

Fig. 9 zeigt eine schematische Darstellung eines vorgeschlagenen Verfahrens gemäß einer weiteren beispielhaften Ausgestaltung der Erfindung.

Dabei zeigt Fig. 9 eine schematische Darstellung eines vorgeschlagenen Verfahrens. Dabei wird das Originalbild zunächst hervorgehoben, beispielsweise den Kontrast oder die Farbsättigung betreffend. Mit einem Schwellwertfilter werden anschließend Reflektionen detektiert. Mittels eines Nachbarschaftsverfahrens werden die Reflektionsbereiche mit den jeweiligen Nachbarpixeln aufgefüllt beziehungsweise übermalt. Ferner wird einen harmonische Interpolation durchgeführt. Ferner wird eine Helligkeitsabhängige Skalierung vorgenommen. Die Reflektionen sind nunmehr vollständig entfernt und die Pupille ist rekonstruiert. Anschließend erfolgt eine Transformation vom RGB-Farbraum in den CIELAB Farbraum. Dort wird der X-Kanals ausgewertet.

Basierend auf einem Entropieverfahrens und dem ausgewerteten X-Kanals, kann ein radiales Integrationsverfahren, ein Intensitätsskalierungsverfahren und/oder ein Kantenfindungsfilter, wie beispielsweise ein Fuzzy-Edge-Detection-Verfahren angewandt werden, um die Pupille sehr präzise erkennen und somit vermessen zu können.

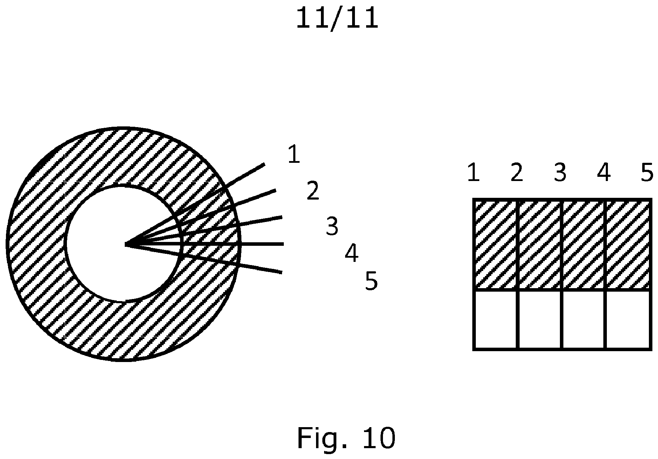

Fig. 10 zeigt eine schematische Darstellung eines vorgeschlagenen Verfahrens gemäß einer weiteren beispielhaften Ausgestaltung der Erfindung.

Dabei zeigt Fig. 10 eine schematische Darstellung eines vorgeschlagenen Verfahrens, bei dem zumindest die Iris und die Pupille in einem rotationssymmetrischen Raum (linke Abbildung) dargestellt werden und anschließend in einen karthesischen Raum (rechte Abbildung) überführt werden. Die Abbildung wird dazu zunächst im rotationssymmetrischen Raum in Segmente unterteilt. Beispielhaft sind in Fig. 10 fünf Segmente in das karthesische System übertragen worden. In dem karthesischen Raum ist nunmehr eine einfache Durchmesserbestimmung möglich, indem eine Durchschnittslänge der gefundenen Kanten der Pupille ermittelt wird. Diese entspricht nunmehr dem Pupillendurchmesser.

Fig. 11 zeigt eine schematische Darstellung einer vorgeschlagenen Vorrichtung gemäß einer weiteren beispielhaften Ausgestaltung der Erfindung.

Dabei zeigt Fig. 11 eine schematische Darstellung einer vorgeschlagenen Vorrichtung zum hochgenauen Bestimmen eines Pupillendurchmessers 270 eines Auges, wobei die Vorrichtung aufweist: Kamerasystem 300 zum räumlichen Erfassen von Objekten. Erfassungsmittel 310, zum bildgebenden Stereoskopen Erfassen 10 einer Augenregion 100 eines Kopfes, mittels des Kamerasystems 300, zum Erzeugen eines ersten und eines zweiten stereoskopen Bildes 110, 111 für ein Auge der Augenregion 100. Transferierungsmittel 311, zum Transferieren 11 der erfassten Augenregion 100 des Kopfes in einen Farbraum 120. Erstes Ermittlungsmittel 317 zum Ermitteln 17 eines Bereiches 130 innerhalb der erfassten Augenregion 100, als einen Bereich 130 innerhalb einer Pupille 200 des Auges der erfassten Augenregion 100. Zweites Ermittlungsmittel 318, zum Ermitteln 18 eines geschlossenen äußeren Randes 140 des Bereiches 130 innerhalb der Pupille 200. Drittes Ermittlungsmittel 320, zum iterativen Ermitteln 20 eines geschlossenen äußeren Pupillenrandes 150.

In Ausführungsformen der Erfindung weist die Vorrichtung weiterhin auf: Erstes Bestimmungsmittel 332, zum Bestimmen 32 eines Punktes 210 in der Pupille 200. Zweites Bestimmungsmittel 334, zum Bestimmen 34 einer ersten Geraden 220 durch den Punkt 210.

Drittes Bestimmungsmittel 336, zum Bestimmen 36 einer, orthogonal zur ersten Geraden 220 angeordneten, zweiten Geraden 230 durch den Punkt 210. Viertes Ermittlungsmittel 338, zum iterativen Ermitteln 38 eines Pupillenmittelpunktes 211.

In Ausführungsformen der Erfindung weist die Vorrichtung weiterhin auf: Erstes Triangulierungsmittel 350, zum triangulierten Überlagern 50 der Pupillenmittelpunkte 211 der Pupillen 200 des Auges der stereoskopen Bilder 110, 111. Zweites Triangulierungsmittel 360, zum triangulierten Überlagern 60 der äußeren Ränder 150 der Pupille 200 des Auges der stereoskopen Bilder 110, 111. Und ein fünftes Ermittlungsmittel 370, zum Ermitteln 70 des Pupillendurchmessers 270, basierend auf den beiden triangulierten Überlagerungen 50, 60. Und dabei ist die Vorrichtung dazu eingerichtet, ein erfindungsgemäßes Verfahren durchzuzuführen.

Die Erfindungsidee kann wie folgt zusammengefasst werden. Es werden ein Verfahren und eine diesbezügliche Vorrichtung bereitgestellt, wodurch es möglich werden kann, mittels eines Stereokamerasystems den Augenbereich eines Probanden aufzunehmen und aus diesen Aufnahmen den Pupillendurchmesser eines jeden Auges sehr präzise bestimmen zu können. Durch die Stereoaufnahme entstehen mehrere zweidimensionale Abbildungen des Augenbereiches. Die aufgenommenen Bilder werden anschließend dahingehend aufbereitet, dass eine darauf folgende computervisualistische Ermittlung des Randes der Pupille eines jeden Auges in jedem Bild vorgenommen werden kann.

Da es bei einer Stereoaufnahme jedoch dazu kommt, dass runde Bereiche elliptisch abgebildet werden, werden die ausgewerteten Einzelbilder anschließend überlagert und unter Berücksichtigung der Position des Stereokamerasystems und des Abstandes zum Probanden, wird der Durchmesser einer jeden einzelnen Pupille dreidimensional äußerst präzise bestimmt.

Ferner kann auch das Auflösungsvermögen des Stereokamerasystems dazu genutzt werden, um den Pupillendurchmesser noch präziser bestimmen zu können.

Die Erfindung betrifft weiterhin einen Datenträger mit darauf gespeicherten Daten oder für die Übersendung über das Internet geeignete, Daten repräsentierende Signalfolge, wobei die Daten ein Programm zum Ablauf auf einem Benutzerrechner als Teil einer Rechneranlage zum hochgenauen Bestimmen eines Pupillendurchmessers 270 eines Auges, mittels eines Kamerasystems 300 zum räumlichen Erfassen von Objekten darstellen, wobei das Programm so ausgebildet ist, dass es beim Ablauf auf dem Benutzerrechner mittel des Kamerasystems 300 eine Augenregion 100 eines Kopfes bildgebend stereoskop erfasst 10, wobei sich für ein Auge der Augenregion 10) ein erstes und ein zweites stereoskopes Bild 110, 111 ergeben, und für ein jedes der beiden stereoskopen Bilder (110, 111) wird die erfasste Augenregion 100 des Kopfes in einen Farbraum 120 durch den Benutzerrechner transferiert 11, wobei anschließend in diesem Farbraum 120 weiterverarbeitet wird. Der Benutzerrechner ermittelt 17 dann einen Bereiches 130 innerhalb der erfassten Augenregion 100, als einen Bereich 130 innerhalb einer Pupille 200 des Auges der erfassten Augenregion 100, wobei der ermittelte Bereich 130 innerhalb der erfassten Augenregion 100 ein Bildkriterium 131 aufweist, wobei das Bildkriterium 131 ein weitgehend homogenes Bildkriterium darstellt. Anschließend ermittelt 18 der Benutzerrechner einen geschlossenen äußeren Rand 140 des Bereiches 130 innerhalb der Pupille 200. Der Benutzerrechner ermittelt iterativ 20 einen geschlossenen äußeren Pupillenrand 150, wobei das iterative Ermitteln 20 des äußeren Pupillenrandes 150 aufweist, dass der äußere Rand 140 durch umlaufendes Ermitteln 22 eines weiteren Bereiches 142 außerhalb des Randes 140, entlang des Randes 140 ermittelt wird, wobei der weitere Bereich 142 an den geschlossenen äußeren Rand 140 des Bereiches 130 innerhalb der Pupille 200 angrenzt. Der Benutzerrechner untersucht iterativ 24 den weiteren Bereich 142 daraufhin, ob der weitere Bereich 142 das Bildkriterium 131 aufweist, und falls zumindest ein Teil des weiteren Bereiches 142 das Bildkriterium 131 aufweist integriert 25 der Benutzerrechner denjenigen Teil des weiteren Bereiches 142, welcher das Bildkriterium 131 aufweist, in den Bereich 130 innerhalb der Pupille 200 des Auges, und der Benutzerrechner weitet (26) den geschlossenen äußeren Rand 140 des Bereiches 130 innerhalb der Pupille 200 auf den integrierten Teil des weiteren Bereiches 142 aus. Falls der weitere Bereich 132 das Bildkriterium 131 hingegen vollständig nicht aufweist, legt der Benutzerrechner den geschlossenen äußeren Rand 140 des Bereiches 130 innerhalb der Pupille 200 als den äußeren Rand 150 der Pupille 200 des Auges fest 28, und der Benutzerrechner ermittelt 30 einen dritten Bereich 160 als Pupille 200 des Auges, wobei der dritte Bereich 160 derjenige Bereich ist, der vollständig vom äußeren Rand 150 der Pupille 200 eingeschlossen ist.

Der Datenträger mit darauf gespeicherten Daten oder für die Übersendung über das Internet geeignete, Daten repräsentierende Signalfolge kann auch auf die weiteren zuvor angegebenen Verfahrensschritte angepasst sein.

Weiterhin kann das verfahren / die Signalfolge auch als Programmlogik implementiert sein-

### Bezugszeichenliste

- 5: Kalibrieren des Kamerasystems
- 10: Bildgebendes stereoskopes Erfassen einer Augenregion eines Kopfes
- 11: Transferieren der Augenregion in einen Farbraum
- 12: Untersuchen der erfassten Augenregion auf Reflektionen
- 13: Entfernen der auftretenden Reflexion
- 14, 15: Durchführen des Verfahrens für ein erstes Auge (14) / zweites Auge (15) der stereoskop-erfassten Augenregion
- 16: Ermitteln eines Auges in der stereoskop-erfassten Augenregion
- 17: Ermitteln eines Bereiches innerhalb der erfassten Augenregion, als Bereich innerhalb einer Pupille des Auges der erfassten Augenregion
- 18: Ermitteln eines geschlossenen äußeren Randes des Bereiches innerhalb der Pupille
- 20: Iteratives Ermitteln des geschlossenen äußeren Pupillenrandes
- 22: Ermitteln eines weiteren Bereiches außerhalb des Randes, entlang des Randes
- 24: Iteratives Untersuchen des weiteren Bereiches daraufhin, ob der weitere Bereich das Bildkriterium aufweist
- 25: Integrieren desjenigen Teiles des weiteren Bereiches, welcher das Bildkriterium aufweist, in den Bereich innerhalb der Pupille
- 26: Ausweiten des geschlossenen äußeren Randes des Bereiches innerhalb der Pupille auf den integrierten Teil des weiteren Bereiches
- 28: Festlegen des geschlossenen äußeren Randes des Bereiches innerhalb der Pupille als den äußeren Rand der Pupille
- 30: Ermitteln eines dritten Bereiches als Pupille
- 32: Bestimmen eines Punktes in der Pupille
- 34: Bestimmen einer ersten Geraden durch den Punkt
- 36: Bestimmen einer zweiten Geraden durch den Punkt
- 38: Iteratives Ermitteln eines Pupillenmittelpunktes
- 40: Ermitteln einer ersten Strecke und einer zweiten Strecke für jede Gerade
- 41: Ermitteln eines ersten Endpunktes entsprechenden Strecke
- 42: Ermitteln eines zweiten Endpunktes der entsprechenden Strecke
- 43: Ermitteln der entsprechenden Strecke, als eine Linie entlang der entsprechenden Geraden von dem entsprechenden ersten Endpunkt ausgehend zu dem entsprechenden zweiten Endpunkt hinführend
- 44: Iteratives längenmäßiges Vergleichen der beiden Strecken der ersten Geraden
- 45: Inkrementieren einer ersten Koordinaten des Punktes in der Pupille, entlang der längeren der beiden Strecken der ersten Geraden
- 46: Inkrementieren einer zweiten Koordinaten des Punktes in der Pupille, entlang der längeren der beiden Strecken der zweiten Geraden
- 47: Festlegen des Punktes in der Pupille als den Mittelpunkt der Pupille
- 50: Trianguliertes Überlagern der beiden Pupillenmittelpunkte der Pupillen des Auges der beiden stereoskopen Bilder,
- 60: Trianguliertes Überlagern der beiden äußeren Ränder der Pupille des Auges der beiden stereoskopen Bilder
- 70: Ermitteln des Pupillendurchmessers, basierend auf den beiden triangulierten Überlagerungen
- 80: Ermitteln eines Größen-Maßstabes
- 100: Augenregion eines Kopfes
- 110: Erstes stereoskopes Bild
- 111: Zweites stereoskopes Bild
- 120: Farbraum
- 130: Bereich innerhalb der Pupille
- 131: Bildkriterium
- 140: Geschlossener äußerer Rand des Bereiches innerhalb der Pupille (abkürzt als: Rand; geschlossener äußerer Rand)
- 142: Weiterer Bereich außerhalb des geschlossenen äußeren Randes des Bereiches innerhalb der Pupille (abgekürzt als: weiterer Bereich)
- 150: Geschlossener äußerer Pupillenrand
- 160: Dritter Bereich als Pupille
- 180: Größen-Maßstab
- 200: Pupille
- 210: Punkt in der Pupille
- 211: Pupillenmittelpunkt
- 212: Erste Koordinate des Punktes in der Pupille
- 213: Zweite Koordinate des Punktes in der Pupille
- 220: Erste Gerade durch den Punkt
- 221: Erster Endpunkt der ersten Strecke der ersten Geraden
- 222: Ersten Strecke der ersten Geraden
- 223: Zweiter Endpunkt der ersten Strecke der ersten Geraden
- 225: Erster Endpunkt der zweiten Strecke der ersten Geraden
- 226: Zweite Strecke der ersten Geraden
- 227: Zweiter Endpunkt der zweiten Strecke der ersten Geraden
- 230: Zweite Gerade durch den Punkt
- 231: Erster Endpunkt der ersten Strecke der zweiten Geraden
- 232: Erste Strecke der zweiten Geraden
- 233: Zweiter Endpunkt der ersten Strecke der zweiten Geraden
- 235: Erster Endpunkt der zweiten Strecke der zweiten Geraden
- 236: Zweite Strecke der zweiten Geraden
- 237: Zweiter Endpunkt der zweiten Strecke der zweiten Geraden
- 240: Pupillenkriterium
- 270: Pupillendurchmesser
- 300: Kamerasystem zum räumlichen Erfassen von Objekten
- 310: Pixelkriterium des Kamerasystems
- 400: Augenmodell
- 420: Pupillenmodell

## Patentansprüche

1. Verfahren zum hochgenauen Bestimmen eines Pupillendurchmessers (270) eines Auges, mittels eines Kamerasystems (300) zum räumlichen Erfassen von Objekten, das Verfahren aufweist:
- Bildgebendes stereoskopes Erfassen (10) einer Augenregion (100) eines Kopfes, mittels des Kamerasystems (300), wobei sich für ein Auge der Augenregion (100) ein erstes und ein zweites stereoskopes Bild (110, 111) ergeben, und
Für ein jedes der beiden stereoskopen Bilder (110, 111) weist das Verfahren ferner auf:
- Transferieren (11) der erfassten Augenregion (100) des Kopfes in einen Farbraum (120), und weiterführen des Verfahrens in diesem Farbraum (120),
- Ermitteln (17) eines Bereiches (130) innerhalb der erfassten Augenregion (100), als einen Bereich (130) innerhalb einer Pupille (200) des Auges der erfassten Augenregion (100), wobei der ermittelte Bereich (130) innerhalb der erfassten Augenregion (100) ein Bildkriterium (131) aufweist, wobei das Bildkriterium (131) ein weitgehend homogenes Bildkriterium darstellt, wobei für ein weitgehend homogenes Bildkriterium eine Bildeigenschaft zumindest in einem signifikant großen Bereich des Bildes Homogenität aufweist,
- Ermitteln (18) eines geschlossenen äußeren Randes (140) des Bereiches (130) innerhalb der Pupille (200),
- Iteratives Ermitteln (20) eines geschlossenen äußeren Pupillenrandes (150), das iterative Ermitteln (20) des äußeren Pupillenrandes (150) aufweisend:
- den äußeren Rand (140) umlaufendes Ermitteln (22) eines weiteren Bereiches (142) außerhalb des Randes (140), entlang des Randes (140), wobei der weitere Bereich (142) an den geschlossenen äußeren Rand (140) des Bereiches (130) innerhalb der Pupille (200) angrenzt,
- Iteratives Untersuchen (24) des weiteren Bereiches (142) daraufhin, ob der weitere Bereich (142) das Bildkriterium (131) aufweist, und
Falls zumindest ein Teil des weiteren Bereiches (142) das Bildkriterium (131) aufweist:
- Integrieren (25) desjenigen Teiles des weiteren Bereiches (142), welcher das Bildkriterium (131) aufweist, in den Bereich (130) innerhalb der Pupille (200) des Auges,
- Ausweiten (26) des geschlossenen äußeren Randes (140) des Bereiches (130) innerhalb der Pupille (200) auf den integrierten Teil des weiteren Bereiches (142), und
Falls der weitere Bereich (132) das Bildkriterium (131) vollständig nicht aufweist:
- Festlegen (28) des geschlossenen äußeren Randes (140) des Bereiches (130) innerhalb der Pupille (200) als den äußeren Rand (150) der Pupille (200) des Auges, und
- Ermitteln (30) eines dritten Bereiches (160) als Pupille (200) des Auges, wobei der dritte Bereich (160) derjenige Bereich ist, der vollständig vom äußeren Rand (150) der Pupille (200) eingeschlossen ist.

2. Verfahren gemäß Anspruch 1, wobei das Verfahren ferner aufweist:
- Bestimmen (32) eines Punktes (210) in der Pupille (200),
- Bestimmen (34) einer ersten Geraden (220) durch den Punkt (210),
- Bestimmen (36) einer zweiten Geraden (230) durch den Punkt (200), wobei die zweite Gerade (230) orthogonal zur ersten Geraden (220) angeordnet ist, und
- Iteratives Ermitteln (38) eines Pupillenmittelpunktes (211), wobei das iterative Ermitteln (38) des Pupillenmittelpunktes (211) aufweist:
- Ermitteln (40) einer ersten Strecke (222, 232) und einer zweiten Strecke (226, 236) für jede Gerade (220, 230), wobei das Ermitteln (40) der jeweiligen Strecke (222, 232, 226, 236) aufweist:
- Ermitteln (41) eines ersten Endpunktes (221, 231, 225, 235) der entsprechenden Strecke (222, 232, 226, 236), wobei der erste Endpunkt (221, 231, 225, 235) dem Punkt (210) in der Pupille (200) entspricht,
- Ermitteln (42) eines zweiten Endpunktes (223, 233, 227, 237) der entsprechenden Strecke (222, 232, 226, 236), wobei der zweite Endpunkt (223, 233, 227, 237) einem Kreuzungspunkt der entsprechenden Geraden (220, 230) mit dem Rand der Pupille (150) entspricht, und
- Ermitteln (43) der entsprechenden Strecke (222, 232, 226, 236), als eine Linie entlang der entsprechenden Geraden (220, 230) von dem entsprechenden ersten Endpunkt (221, 231, 225, 235) ausgehend zu dem entsprechenden zweiten Endpunkt (223, 233, 227, 237) hinführend,
- Iteratives längenmäßiges Vergleichen (44) der beiden Strecken (222, 226) der ersten Geraden (220), und
Falls die beiden Strecken (222, 226) der ersten Geraden (220) nicht gleich lang sind:
- Inkrementieren (45) einer ersten Koordinaten (212) des Punktes (210) in der Pupille (200), entlang der längeren der beiden Strecken (222, 226) der ersten Geraden (220), und
Falls die beiden Strecken (232, 236) der zweiten Geraden (230) nicht gleich lang sind:
- Inkrementieren (46) einer zweiten Koordinaten (213) des Punktes in der Pupille (200), entlang der längeren der beiden Strecken (232, 236) der zweiten Geraden (230), und
Falls die beiden Strecken (222, 226) der ersten Geraden (220) gleichlang sind und die beiden Strecken (232, 236) der zweiten Geraden (230) gleichlang sind:
- Festlegen (47) des Punktes (210) in der Pupille (200) als den Pupillenmittelpunkt (211) der Pupille (200) des Auges.

3. Verfahren gemäß Anspruch 2, wobei das Verfahren ferner aufweist:-
Trianguliertes Überlagern (50) der beiden Mittelpunkte (211) der Pupillen (200) des Auges der beiden stereoskopen Bilder (110, 111),
- Trianguliertes Überlagern (60) der beiden äußeren Ränder (150) der Pupille (200) des Auges der beiden stereoskopen Bilder (110, 111), und
- Ermitteln (70) des Pupillendurchmessers (270), basierend auf den beiden triangulierten Überlagerungen (50, 60).

4. Verfahren gemäß einem der vorhergehenden Ansprüche, wobei das Verfahren ferner aufweist:
- Kalibrieren (5) des Kamerasystems (300) zum räumlichen Erfassen von Objekten, vor dem bildgebenden stereoskopen Erfassen (10) der Augenregion (100) des Kopfes, wobei das Kalibrieren (5) vorzugsweise basierend auf einem vordefinierten Winkel und/oder einem vordefinierten Abstand des Kamerasystems (300) zu einem Bezugsobjekt erfolgt, und wobei der Kopf der zu erfassenden Augenregion (100), sich während der stereoskopen Erfassung (10) an der Position des Bezugsobjektes befindet.

5. Verfahren gemäß einem der vorhergehenden Ansprüche, wobei das Verfahren ferner aufweist:
- Ermitteln (13) eines Auges in der stereoskop-erfassten (10) Augenregion (100), basierend auf einem Abgleich der stereoskop-erfassten (10) Augenregion (100) mit einem Augenmodell (400).

6. Verfahren gemäß einem der vorhergehenden Ansprüche, wobei
der Farbraum (120) einem Farbraum entspricht, in welchem das Bildkriterium (131) besonders geeignet ermittelbar ist.

7. Verfahren gemäß einem der vorhergehenden Ansprüche, wobei
das Bildkriterium (131) ein Kontrastkriterium aufweist.

8. Verfahren gemäß Anspruch 7, wobei
der Farbraum (120) einem CIELAB Farbraum entspricht.

9. Verfahren gemäß einem der vorhergehenden Ansprüche, wobei
das Ermitteln (17) des Bereiches (130) innerhalb der erfassten Augenregion (100), als Bereich (130) innerhalb der Pupille (200) des Auges der erfassten Augenregion (100), basierend auf einem Pupillenkriterium (220) erfolgt, vorzugsweise basierend auf einem Abgleich mit einem Pupillenmodell (420) erfolgt.

10. Verfahren gemäß einem der vorhergehenden Ansprüche, wobei das Verfahren ferner aufweist:
- Ermitteln (80) eines Größen-Maßstabes (180), basierend auf einer Entfernung des Kamerasystems (300) zu der stereoskop-erfassten (10) Augenregion (100) des Kopfes und eines Pixelkriteriums (310) des Kamerasystems (300), und wobei
das Ermitteln (70) des jeweiligen Pupillendurchmessers (270) basierend auf dem ermittelten Größen-Maßstab (180) erfolgt.

11. Verfahren gemäß Anspruch 10, wobei
das Pixelkriterium (310) des Kamerasystems (300) eine Pixelgröße beziehungsweise Pixelbreite und eine Pixelanzahl aufweist.

12. Verfahren gemäß einem der vorhergehenden Ansprüche, wobei das Verfahren ferner, vor dem Ermitteln (17) des Bereiches (130) innerhalb der erfassten Augenregion (100), als den Bereich (130) innerhalb der Pupille (200) des Auges der erfassten Augenregion (100), aufweist:
- Untersuchen (12) der erfassten Augenregion (100) auf Reflektionen, und
falls die erfasste Augenregion (100) mindestens eine Reflexion aufweist:
- Entfernen (13) der auftretenden Reflexion beziehungsweise Reflexionen mittels eines Nachbarschaftskriteriums, derart, dass eine Bildinformation im einem Bereich in welchem die Reflexion auftritt, mit entsprechenden Bildinformationen der auf dem Nachbarschaftskriterium basierenden entsprechenden Nachbarpixel ersetzt werden.

13. Verfahren gemäß einem der vorhergehenden Ansprüche, wobei das Verfahren ferner aufweist:
- Durchführen des Verfahrens (14) für ein erstes Auge der stereoskop-erfassten (10) Augenregion (100), und
- Durchführen des Verfahrens (15) für ein zweites Auge der stereoskop-erfassten (10) Augenregion (100).

14. Vorrichtung zum hochgenauen Bestimmen eines Pupillendurchmessers (270) eines Auges, wobei die Vorrichtung aufweist:
- Kamerasystem (300) zum räumlichen Erfassen von Objekten,
- Erfassungsmittel (310), zum bildgebenden Stereoskopen Erfassen (10) einer Augenregion (100) eines Kopfes, mittels des Kamerasystems (300), zum Erzeugen eines ersten und eines zweiten stereoskopen Bildes (110, 111) für ein Auge der Augenregion (100),
- Transferierungsmittel (311), zum Transferieren (11) der erfassten Augenregion (100) des Kopfes in einen Farbraum (120),
- Erstes Ermittlungsmittel (317) zum Ermitteln (17) eines Bereiches (130) innerhalb der erfassten Augenregion (100), als einen Bereich (130) innerhalb einer Pupille (200) des Auges der erfassten Augenregion (100),
- Zweites Ermittlungsmittel (318), zum Ermitteln (18) eines geschlossenen äußeren Randes (140) des Bereiches (130) innerhalb der Pupille (200),
- Drittes Ermittlungsmittel (320), zum iterativen Ermitteln (20) eines geschlossenen äußeren Pupillenrandes (150),
und wobei
die Vorrichtung dazu eingerichtet ist, das Verfahren gemäß irgendeinem der vorhergehenden Ansprüche durchzuzuführen.

15. Vorrichtung gemäß Anspruch 14, weiterhin aufweisend:
- Erstes Bestimmungsmittel (332), zum Bestimmen (32) eines Punktes (210) in der Pupille (200),
- Zweites Bestimmungsmittel (334), zum Bestimmen (34) einer ersten Geraden (220) durch den Punkt (210),
- Drittes Bestimmungsmittel (336), zum Bestimmen (36) einer, orthogonal zur ersten Geraden (220) angeordneten, zweiten Geraden (230) durch den Punkt (210),
- Viertes Ermittlungsmittel (338), zum iterativen Ermitteln (38) eines Pupillenmittelpunktes (211),

16. Vorrichtung gemäß Anspruch 15, weiterhin aufweisend:
- Erstes Triangulierungsmittel (350), zum triangulierten Überlagern (50) der Pupillenmittelpunkte (211) der Pupillen (200) des Auges der stereoskopen Bilder (110, 111),
- Zweites Triangulierungsmittel (360), zum triangulierten Überlagern (60) der äußeren Ränder (150) der Pupille (200) des Auges der stereoskopen Bilder (110, 111), und
- Fünftes Ermittlungsmittel (370), zum Ermitteln (70) des Pupillendurchmessers (270), basierend auf den beiden triangulierten Überlagerungen (50, 60).

17. Computerprogrammprodukt, umfassend Befehle, die bewirken, dass die Vorrichtung gemäß einem der vorhergehenden Ansprüche 14 - 16 Vorrichtung ein Verfahren gemäß irgendeinem der Ansprüche 1 bis 13 ausführt.

18. Datenträger, aufweisend ein Computerprogrammprodukt gemäß Anspruch 17.

## Claims

1. A method for highly accurate determination of a pupil diameter (270) of an eye, by means of a camera system (300) for spatial detection of objects, the method comprising:
- Imaging stereoscopically (10) a region of an eye (100) of a head, using the camera system (300), resulting in first and second stereoscopic images (110, 111) for an eye of the region of the eye (100); and
for each of the two stereoscopic images (110, 111), the method further comprises:
- transferring (11) the captured eye region (100) of the head into a color space (120), and continuing the method in said color space (120),
- determining (17) an area (130) within the sensed eye region (100), as an area (130) within a pupil (200) of the eye of the sensed eye region (100), the determined area (130) within the sensed eye region (100) having an image criterion (131), wherein the image criterion (131) represents a substantially homogeneous image criterion, wherein for a substantially homogeneous image criterion an image property has homogeneity at least in a significantly large area of the image,
- determining (18) a closed outer edge (140) of the area (130) within the pupil (200),
- iteratively determining (20) a closed outer pupil margin (150), comprising iteratively determining (20) the outer pupil margin (150):
- circumferentially determining (22) the outer rim (140) a further area (142) outside the rim (140), along the rim (140), the further area (142) being adjacent to the closed outer rim (140) of the area (130) inside the pupil (200),
- iteratively examining (24) the further region (142) to determine whether the further region (142) exhibits the image criterion (131), and
if at least a part of the further area (142) has the image criterion (131):
- Integrating (25) that part of the further area (142) which has the image criterion (131) into the area (130) inside the pupil (200) of the eye,
- extending (26) the closed outer edge (140) of the area (130) within the pupil (200) to the integrated part of the further area (142), and
if the further area (132) completely lacks the image criterion (131):
- determining (28) the closed outer edge (140) of the area (130) within the pupil (200) as the outer edge (150) of the pupil (200) of the eye, and
- determining (30) a third area (160) as the pupil (200) of the eye, wherein the third area (160) is the area completely enclosed by the outer edge (150) of the pupil (200).

2. The method according to claim 1, the method further comprising:
- determining (32) a point (210) in the pupil (200),
- determining (34) a first straight line (220) through the point (210),
- determining (36) a second straight line (230) through the point (200), the second straight line (230) being orthogonal to the first straight line (220), and
- iteratively determining (38) a pupil center point (211), the iteratively determining (38) the pupil center point (211) comprising:
- determining (40) a first distance (222, 232) and a second distance (226, 236) for each straight line (220, 230), wherein determining (40) the respective distance (222, 232, 226, 236) comprises:
- determining (41) a first end point (221, 231, 225, 235) of the respective line (222, 232, 226, 236), wherein the first end point (221, 231, 225, 235) corresponds to the point (210) in the pupil (200),
- determining (42) a second end point (223, 233, 227, 237) of the corresponding line (222, 232, 226, 236), the second end point (223, 233, 227, 237) corresponding to a point of intersection of the corresponding line (220, 230) with the edge of the pupil (150), and
- determining (43) the corresponding distance (222, 232, 226, 236) as a line along the corresponding straight line (220, 230) from the corresponding first end point (221, 231, 225, 235) leading to the corresponding second end point (223, 233, 227, 237),
- iteratively comparing (44) in length the two lines (222, 226) of the first straight line (220), and
If the two stretches (222, 226) of the first straight line (220) are not of equal length:
- incrementing (45) a first coordinate (212) of the point (210) in the pupil (200), along the longer of the two distances (222, 226) of the first straight line (220), and
If the two distances (232, 236) of the second straight line (230) are not of equal length:
- incrementing (46) a second coordinate (213) of the point in the pupil (200), along the longer of the two lines (232, 236) of the second straight line (230), and
If the two lines (222, 226) of the first straight line (220) are of equal length and the two lines (232, 236) of the second straight line (230) are of equal length:
- establishing (47) the point (210) in the pupil (200) as the pupil center (211) of the pupil (200) of the eye.

3. The method according to claim 2, wherein the method further comprises:
- triangulated superimposition (50) of the two centers (211) of the pupils (200) of the eye of the two stereoscopic images (110, 111),
- triangulating (60) the two outer edges (150) of the pupil (200) of the eye of the two stereoscopic images (110, 111), and
- determining (70) the pupil diameter (270) based on the two triangulated overlays (50, 60).

4. The method according to any one of the preceding claims, the method further comprising:
- calibrating (5) the camera system (300) for spatially capturing objects, prior to the stereoscopic imaging capture (10) of the eye region (100) of the head, wherein the calibrating (5) is preferably based on a predefined angle and/or a predefined distance of the camera system (300) to a reference object, and wherein the head of the eye region (100) to be captured, is located at the position of the reference object during the stereoscopic capture (10).

5. The method according to any one of the preceding claims, wherein the method further comprises:
- determining (13) an eye in the stereoscopically captured (10) eye region (100) based on matching the stereoscopically captured (10) eye region (100) with an eye model (400).

6. The method according to any one of the preceding claims, wherein the color space (120) corresponds to a color space in which the image criterion (131) is particularly suitably determinable.

7. The method according to any one of the preceding claims, wherein the image criterion (131) comprises a contrast criterion.

8. The method according to claim 7, wherein the color space (120) corresponds to a CIELAB color space.

9. The method according to any one of the preceding claims, wherein
determining (17) the area (130) within the detected eye region (100), as an area (130) within the pupil (200) of the eye of the detected eye region (100), based on a pupil criterion (220) is performed, preferably based on a matching with a pupil model (420).

10. The method according to any one of the preceding claims, wherein the method further comprises:
- determining (80) a size scale (180) based on a distance of the camera system (300) to the stereoscopically captured (10) eye region (100) of the head and a pixel criterion (310) of the camera system (300), and wherein
determining (70) the respective pupil diameter (270) is based on the determined size scale (180).

11. The method according to claim 10, wherein
the pixel criterion (310) of the camera system (300) comprises a pixel size or pixel width and a pixel number.

12. The method according to any one of the preceding claims, wherein the method further comprises, prior to determining (17) the area (130) within the captured eye region (100), as the area (130) within the pupil (200) of the eye of the captured eye region (100):
- examining (12) the sensed eye region (100) for reflections; and
if the detected eye region (100) has at least one reflection:
- removing (13) the occurring reflection(s) by means of a neighborhood criterion, such that image information in a region in which the reflection occurs is replaced with corresponding image information of the corresponding neighbor pixels based on the neighborhood criterion.

13. The method according to any one of the preceding claims, the method further comprising:
- performing the method (14) for a first eye of the stereoscopically captured (10) eye region (100); and
- performing the method (15) for a second eye of the stereoscopically captured (10) eye region (100).

14. Apparatus for highly accurately determining a pupil diameter (270) of an eye, the apparatus comprising:
- camera system (300) for spatially detecting objects,
- acquisition means (310), for imaging stereoscopic acquisition (10) of an eye region (100) of a head, by means of the camera system (300), for generating a first and a second stereoscopic image (110, 111) for an eye of the eye region (100),
- transfer means (311), for transferring (11) the captured eye region (100) of the head into a color space (120),
- first detecting means (317) for detecting (17) an area (130) within the detected eye region (100), as an area (130) within a pupil (200) of the eye of the detected eye region (100),
- second detecting means (318), for detecting (18) a closed outer edge (140) of the area (130) within the pupil (200),
- third determining means (320), for iteratively determining (20) a closed outer pupil margin (150),
and wherein
the apparatus is adapted to perform the method according to any one of the preceding claims.

15. The apparatus according to claim 14, further comprising:
- first determining means (332), for determining (32) a point (210) in the pupil (200),
- second determining means (334), for determining (34) a first straight line (220) through the point (210),
- third determining means (336), for determining (36) a second straight line (230), arranged orthogonally to the first straight line (220), through the point (210),
- fourth determining means (338), for iteratively determining (38) a pupil center point (211).

16. The apparatus according to claim 15, further comprising:
- first triangulation means (350), for triangulating (50) the pupil centers (211) of the pupils (200) of the eye of the stereoscopic images (110, 111),
- second triangulation means (360), for triangulated superposition (60) of the outer edges (150) of the pupil (200) of the eye of the stereoscopic images (110, 111), and
- fifth determining means (370), for determining (70) the pupil diameter (270) based on the two triangulated overlays (50, 60).

17. Computer program product comprising instructions causing the apparatus according to any of the preceding claims 14-16 to perform a method according to any of claims 1-13.

18. Data carrier comprising a computer program product according to claim 17.

## Revendications

1. Procédé pour déterminer avec une grande précision le diamètre de la pupille (270) d'un oeil, au moyen d'un système de caméra (300) pour la détection spatiale d'objets, le procédé comprenant :
- la saisie stéréoscopique d'images (10) d'une région oculaire (100) d'une tête, au moyen du système de caméra (300), une première et une deuxième image stéréoscopique (110, 111) étant obtenues pour un oeil de la région oculaire (100), et
Pour chacune des deux images stéréoscopiques (110, 111), le procédé comprend en outre :
- le transfert (11) de la région oculaire (100) de la tête détectée dans un espace chromatique (120), et la poursuite du procédé dans cet espace chromatique (120),
- détermination (17) d'une zone (130) à l'intérieur de la région oculaire détectée (100), en tant que zone (130) à l'intérieur d'une pupille (200) de oeil de la région oculaire détectée (100), la zone déterminée (130) à l'intérieur de la région oculaire détectée (100) présentant un critère d'image (131), le critère d'image (131) représentant un critère d'image largement homogène, une propriété d'image présentant, pour un critère d'image largement homogène, une homogénéité au moins dans une zone significativement grande de l'image,
- détermination (18) d'un bord extérieur fermé (140) de la zone (130) à l'intérieur de la pupille (200),
- la détermination itérative (20) d'un bord extérieur fermé de pupille (150), la détermination itérative (20) du bord extérieur de pupille (150) comprenant :
- la détermination (22) d'une autre zone (142) à l'extérieur du bord (140), le long du bord (140), entourant le bord extérieur (140), l'autre zone (142) étant adjacente au bord extérieur fermé (140) de la zone (130) à l'intérieur de la pupille (200),
- examiner (24) de manière itérative l'autre zone (142) pour déterminer si l'autre zone (142) présente le critère d'image (131), et
Si au moins une partie de la zone supplémentaire (142) présente le critère d'image (131) :
- intégrer (25) la partie de l'autre zone (142) qui présente le critère d'image (131) dans la zone (130) à l'intérieur de la pupille (200) de oeil,
- l'extension (26) du bord extérieur fermé (140) de la zone (130) à l'intérieur de la pupille (200) à la partie intégrée de l'autre zone (142), et
Si l'autre zone (132) ne présente pas complètement le critère d'image (131) :
- déterminer (28) le bord extérieur fermé (140) de la zone (130) à l'intérieur de la pupille (200) comme étant le bord extérieur (150) de la pupille (200) de l'oeil, et
- détermination (30) d'une troisième zone (160) en tant que pupille (200) de oeil, la troisième zone (160) étant la zone entièrement incluse dans le bord extérieur (150) de la pupille (200).

2. Le procédé selon la revendication 1, le procédé comprenant en outre :
- détermination (32) d'un point (210) dans la pupille (200),
- détermination (34) d'une première ligne droite (220) passant par le point (210),
- détermination (36) d'une seconde droite (230) passant par le point (200), la seconde droite (230) étant orthogonale à la première droite (220), et
- déterminer de manière itérative (38) un centre de pupille (211), la détermination itérative (38) du centre de pupille (211) comprenant :
- détermination (40) d'une première distance (222, 232) et d'une deuxième distance (226, 236) pour chaque ligne droite (220, 230), la détermination (40) de la distance respective (222, 232, 226, 236) comprenant :
- détermination (41) d'un premier point d'extrémité (221, 231, 225, 235) du trajet correspondant (222, 232, 226, 236), le premier point d'extrémité (221, 231, 225, 235) correspondant au point (210) dans la pupille (200),
- détermination (42) d'un deuxième point d'extrémité (223, 233, 227, 237) du trajet correspondant (222, 232, 226, 236), le deuxième point d'extrémité (223, 233, 227, 237) correspondant à un point d'intersection de la ligne droite correspondante (220, 230) avec le bord de la pupille (150), et
- détermination (43) de la distance correspondante (222, 232, 226, 236) comme une ligne le long de la ligne droite correspondante (220, 230) en partant du premier point d'extrémité correspondant (221, 231, 225, 235) et en allant vers le second point d'extrémité correspondant (223, 233, 227, 237),
- comparer itérativement (44) la longueur des deux segments (222, 226) de la première droite (220), et
Si les deux segments (222, 226) de la première ligne droite (220) n'ont pas la même longueur :
- incrémenter (45) une première coordonnée (212) du point (210) dans la pupille (200), le long du plus long des deux segments (222, 226) de la première ligne droite (220), et
Si les deux segments (232, 236) de la deuxième droite (230) n'ont pas la même longueur :
- incrémenter (46) une deuxième coordonnée (213) du point dans la pupille (200), le long du plus long des deux segments (232, 236) de la deuxième droite (230), et
Si les deux segments (222, 226) de la première droite (220) sont de même longueur et les deux segments (232, 236) de la deuxième droite (230) sont de même longueur :
- détermination (47) du point (210) dans la pupille (200) comme étant le centre pupillaire (21 1 ) de la pupille (200) de oeil.

3. Le procédé selon la revendication 2, ledit procédé comprenant en outre :
- superposition triangulée (50) des deux centres (211) des pupilles (200) de l'oeil des deux images stéréoscopiques (110, 111),
- superposition triangulée (60) des deux bords extérieurs (150) de la pupille (200) de l'oeil des deux images stéréoscopiques (110, 111), et
- détermination (70) du diamètre de la pupille (270) sur la base des deux superpositions triangulées (50, 60).

4. Le procédé selon l'une quelconque des revendications précédentes, ledit procédé comprenant en outre :
- calibrage (5) du système de caméra (300) pour la saisie spatiale d'objets, avant la saisie stéréoscopique (10) de la région oculaire (100) de la tête, le calibrage (5) étant de préférence basé sur un angle prédéfini et/ou une distance prédéfinie du système de caméra (300) par rapport à un objet de référence, et la tête de la région oculaire (100) à saisir se trouvant à la position de l'objet de référence pendant la saisie stéréoscopique (10).

5. Le procédé selon l'une quelconque des revendications précédentes, le procédé comprenant en outre :
- Détermination (13) d'un oeil dans la région oculaire (100) acquise par stéréoscopie (10), sur la base d'une comparaison de la région oculaire (100) acquise par stéréoscopie (10) avec un modèle oculaire (400).

6. Le procédé selon l'une des revendications précédentes, dans lequel l'espace chromatique (120) correspond à un espace chromatique dans lequel le critère d'image (131) peut être déterminé de manière particulièrement appropriée.

7. Le procédé selon l'une quelconque des revendications précédentes, dans lequel le critère d'image (131) comprend un critère de contraste.

8. Le procédé selon la revendication 7, dans lequel l'espace colorimétrique (120) correspond à un espace colorimétrique CIELAB.

9. Le procédé selon l'une quelconque des revendications précédentes, dans lequel la détermination (17) de la zone (130) à l'intérieur de la région oculaire (100) détectée, en tant que zone (130) à l'intérieur de la pupille (200) de l'oeil de la région oculaire (100) détectée, est effectuée sur la base d'un critère de pupille (220), de préférence sur la base d'une comparaison avec un modèle de pupille (420).

10. Le procédé selon l'une quelconque des revendications précédentes, ledit procédé comprenant en outre :
- détermination (80) d'une échelle de taille (180) basée sur une distance du système de caméra (300) à la région oculaire (100) de la tête détectée par stéréoscopie (10) et un critère de pixel (310) du système de caméra (300), et dans lequel
la détermination (70) du diamètre de pupille respectif (270) s'effectue sur la base de l'échelle de taille déterminée (180).

11. Le procédé selon la revendication 10, dans lequel
le critère de pixel (310) du système de caméra (300) comprend une taille de pixel ou une largeur de pixel et un nombre de pixels.

12. Le procédé selon l'une quelconque des revendications précédentes, le procédé comprenant en outre, avant la détermination (17) de la zone (130) à l'intérieur de la région oculaire (100) détectée, la zone (130) à l'intérieur de la pupille (200) de l'oeil de la région oculaire (100) détectée :
- l'examen (12) de la région oculaire détectée (100) pour détecter des réflexions, et
si la région oculaire (100) détectée présente au moins un reflet :
- suppression (13) de la réflexion ou des réflexions apparaissant au moyen d'un critère de voisinage, de telle sorte qu'une information d'image dans une zone dans laquelle la réflexion apparaît est remplacée par des informations d'image correspondantes des pixels voisins correspondants basés sur le critère de voisinage.

13. Le procédé selon l'une quelconque des revendications précédentes, ledit procédé comprenant en outre :
- la mise en oeuvre du procédé (14) pour un premier oeil de la région oculaire (100) détectée par stéréoscopie (10), et
- la mise en oeuvre du procédé (15) pour un deuxième oeil de la région oculaire (10) détectée par stéréoscopie (100).

14. Dispositif pour déterminer avec une grande précision le diamètre de la pupille (270) d'un oeil, le dispositif comprenant :
- système de caméra (300) pour la détection spatiale d'objets,
- des moyens d'acquisition (310) pour l'acquisition stéréoscopique (10) par imagerie d'une région oculaire (100) d'une tête, au moyen du système de caméra (300), pour générer une première et une deuxième image stéréoscopique (110, 111) pour un oeil de la région oculaire (100),
- des moyens de transfert (311), pour transférer (11) la région oculaire détectée (100) de la tête dans un espace couleur (120),
- premier moyen de détermination (317) pour déterminer (17) une zone (130) à l'intérieur de la région oculaire détectée (100), en tant que zone (130) à l'intérieur d'une pupille (200) de l'oeil de la région oculaire détectée (100),
- deuxième moyen de détection (318) pour détecter (18) un bord extérieur fermé (140) de la zone (130) à l'intérieur de la pupille (200),
- troisième moyen de détermination (320), pour déterminer de manière itérative (20) un bord extérieur fermé de la pupille (150),
et dans lequel
le dispositif est adapté pour mettre en oeuvre le procédé selon l'une quelconque des revendications précédentes.

15. Le dispositif selon la revendication 14, comprenant en outre :
- premier moyen de détermination (332) pour déterminer (32) un point (210) dans la pupille (200),
- deuxième moyen de détermination (334), pour déterminer (34) une première ligne droite (220) passant par le point (210),
- troisième moyen de détermination (336), pour déterminer (36) une deuxième droite (230), orthogonale à la première droite (220), passant par le point (210),
- quatrième moyen de détermination (338), pour déterminer (38) de manière itérative un centre de pupille (211),

16. Le dispositif selon la revendication 15, comprenant en outre :
- premier moyen de triangulation (350), pour la superposition triangulée (50) des centres de pupille (211) des pupilles (200) de l'oeil des images stéréoscopiques (110, 111),
- deuxième moyen de triangulation (360), pour la superposition triangulée (60) des bords extérieurs (150) de la pupille (200) de l'oeil des images stéréoscopiques (110, 111), et
- cinquième moyen de détermination (370), pour déterminer (70) le diamètre de la pupille (270), sur la base des deux superpositions triangulées (50, 60).

17. Produit programme d'ordinateur comprenant des instructions pour que le dispositif selon l'une quelconque des revendications précédentes 14 à 16 exécute un procédé selon l'une quelconque des revendications 1 à 13.

18. Support de données comprenant un produit programme d'ordinateur selon la revendication 17.
